# EUROPÄISCHE PATENTSCHRIFT

(11) **EP 0 728 741 B1**
(45) Veröffentlichungstag und Bekanntmachung des Hinweises auf die Patenterteilung: **20.09.2000**
(21) Anmeldenummer: 96102255.5
(22) Anmeldetag: 15.02.1996
(51) Int. Cl.: C07C 311/58, C07C 335/42, C07D 303/48, C07D 331/02, C07D 203/08, A61K 31/64

(54) **Substituierte Benzolsulfonylharnstoffe und -thioharnstoffe, Verfahren zu ihrer Herstellung, ihre Verwendung zur Herstellung pharmazeutischer Präparate und sie enthaltende Medikamente**
Substituted benzenesulfonylureas and -thioureas, process for their preparation, their use in the production of pharmaceutical preparations and medicaments containing them
Benzènesulfonylurées et -thiourées substituées, procédé pour leur préparation, leur utilisation dans la production de préparations pharmaceutiques et médicaments les contenant

(30) Priorität: 21.02.1995 DE 19505910
(43) Veröffentlichungstag der Anmeldung: 28.08.1996
(73) Patentinhaber: HOECHST AKTIENGESELLSCHAFT, 65929 Frankfurt am Main (DE)
(72) Erfinder: Englert, Heinrich, Dr., D-65719 Hofheim (DE); Gerlach, Uwe, Dr., D-65795 Hattersheim (DE); Mania, Dieter, Dr, D-61462 Königstein (DE); Gögelein, Heinz, Dr., D-60259 Frankfurt (DE); Kaiser, Joachim, Dr., D-60431 Frankfurt (DE)

(56) Entgegenhaltungen:
- EP-A- 0 612 724
- BE-A- 754 454
- DE-B- 1 198 354

## Beschreibung

Die Erfindung betrifft substituierte Benzolsulfonylharnstoffe und -thioharnstoffe der Formel I 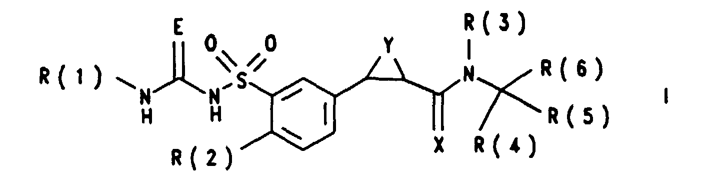 worin bedeuten:
- R(1): Wasserstoff, Alkyl mit 1, 2, 3, 4, 5, 6 oder 7 C-Atomen, Cycloalkyl mit 3, 4, 5, 6 oder 7 C-Atomen;
- R(2): Wasserstoff, F, Cl, Cycloalkyl mit 3, 4, 5 oder 6 C-Atomen oder eine C_{b}H_{2b+1}-Gruppe, in der 1, 2, 3 oder 4 C-Atome durch Heteroatome, z.B. O, N, S, ersetzt sein können;
b 1, 2, 3, 4, 5, 6, 7 oder 8;
- R(3): H, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch Heteroatome, z.B. 0, NH, S, ersetzt sind;
a 1, 2, 3, 4, 5, 6, 7, 8, 9 oder 10;
- R(4): H, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch Heteroatome, z.B. 0, NH oder S, ersetzt sind;
a 1, 2, 3, 4, 5, 6, 7, 8, 9 oder 10;
oder
- R(3) und R(4): gemeinsam eine C_{b}H_{2b}-Gruppe, in der eine oder mehrere der CH₂-Gruppen durch Heteroatome, z. B. durch O, S oder NH, ersetzt sein können;
b 1, 2, 3, 4, 5, 6, 7 oder 8;
- R(5): H, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe in der 1, 2, 3 oder 4 C-Atome durch Heteroatome, z.B. O, N oder S, ersetzt sind;
a 1, 2, 3, 4, 5, 6, 7, 8, 9 oder 10;
- R(6): H, Aryl, Alkyl mit 1, 2, 3, 4, 5, 6, 7, 8, 9, 10, 11 oder 12 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch Heteroatome, z.B. O, NH, S, ersetzt sind;
a 1, 2, 3, 4, 5, 6, 7, 8, 9 oder 10;
- E: Sauerstoff oder Schwefel;
- X: Sauerstoff oder Schwefel;
- Y: ICR(7)₂]₁₋₄, O, S oder NH;
R(7) Wasserstoff, Alkyl mit 1, 2, 3 oder 4 C-Atomen, F, Cl oder Br;
- Aryl: Phenyl, Thienyl, Furyl, Pyrrolyl, Thiazolyl, Naphthyl oder Pyridyl;
sowie ihre pharmazeutisch verträglichen Salze.

Der Begriff Alkyl beschreibt, sofern nicht anders angegeben, geradkettige oder verzweigte gesättigte Kohlenwasserstoffreste. Der Cycloalkylrest kann zusätzlich einen Alkylsubstituenten tragen. Als Halogensubstituent sind die Elemente Fluor, Chlor, Brom und lod einsetzbar.

Die für Aryl stehenden aromatischen Systeme Phenyl, Thienyl, Furyl, Pyrrolyl, Thiazolyl, Naphthyl, Pyridyl können jeweils durch ein bis drei Substituenten wie Alkyl mit 1 oder 2 C-Atomen, Alkoxy mit 1 oder 2 C-Atomen, Cl, Br, F, substituiert sein. Insbesondere steht Aryl für Phenyl, Naphthyl und Thienyl. Weiterhin können Verbindungen mit Chiralitätszentren, etwa in den Alkylketten Y, R(3), R(4), R(5) und R(6) auftreten. In diesem Fall gehören sowohl die einzelnen Antipoden für sich, als auch eine Mischung der beiden Enantiomere in unterschiedlichen Verhältnissen, sowie die dazugehörigen Mesoverbindungen oder Mischungen aus Mesoverbindungen, den Enantiomeren oder Diastereomeren zur Erfindung.

Ähnliche Sulfonylharnstoffe mit blutzuckersenkender Wirkung sind aus der belgischen Anmeldung BE 754454 und der deutschen Offenlegungsschrift 1 198 354 bekannt. In den deutschen Patentanmeldungen P 43 41 655.1 (HOE 93/F 399) und P 43 44 957.3 (HOE 93/F 439) werden Benzolsulfonylharnstoffe und -thioharnstoffe vorgeschlagen, die aber keine Cyclo-Gruppierung entsprechend dem Y enthaltenden Ring aufweisen.

Dort werden die blutzuckersenkenden Wirkungen der Sulfonylharnstoffe beschrieben. Als Prototyp solcher blutzuckersenkenden Sulfonylharnstoffe gilt das Glibenclamid, das als Mittel zur Behandlung des Diabetes mellitus therapeutisch verwendet wird und in der Wissenschaft ein vielbeachtetes Werkzeug zur Erforschung sogenannter ATP sensitiver Kaliumkanäle dient. Neben seiner blutzuckersenkenden Wirkung besitzt das Glibenclamid noch andere Wirkungen, die bislang therapeutisch noch nicht eingesetzt werden können, die aber allesamt auf Blockade eben dieser ATP-sensitiven KaliumKanäle zurückgeführt werden. Dazu gehört insbesondere eine antifibrillatorische Wirkung am Herzen. Bei der Behandlung des Kammerflimmerns oder seiner Vorstufen wäre jedoch eine gleichzeitige Blutzuckersenkung unerwünscht oder gar gefährlich, da sie den Zustand des Patienten weiter verschlechtern kann.

In der Patentanmeldung EP-A-612 724 werden bestimmte Benzolsulfonylharnstoffe und -thioharnstoffe beschrieben, die den Blutzucker deutlich geringer beeinflussen als Glibenclamid, die aber auch keine Cyclo-Gruppierung entsprechend dem Y enthaltenden Ring aufweisen.

Aufgabe der vorliegenden Erfindung war es, weitere Verbindungen zu synthetisieren, die eine gleich gute Herzwirkung wie Glibenclamid aufweisen, aber den Blutzucker in herzwirksamen Dosen oder Konzentrationen nicht oder deutlich geringer beeinflussen als Glibenclamid.

Als Versuchstiere zum Nachweis solcher Wirkungen eignen sich zum Beispiel Mäuse, Ratten, Meerschweinchen, Kaninchen, Hunde, Affen oder Schweine.

Die Verbindungen I dienen als Arzneimittelwirkstoffe in der Human- und Veterinärmedizin. Ferner können sie als Zwischenprodukte zur Herstellung weiterer Arzneimittelwirkstoffe verwendet werden.

In den folgenden bevorzugten Verbindungen können die Reste Cycloalkyl und Aryl, wie bereits auf den Seiten 2 und 3 angegeben, substituiert sein.

Bevorzugt sind die Verbindungen I, in denen bedeuten:
- R(1): Wasserstoff, Alkyl mit 1, 2, 3, 4, 5, 6 oder 7 C-Atomen oder Cycloalkyl mit 3, 4, 5, 6 oder 7 C-Atomen,
- R(2): Wasserstoff, F, Cl, Cycloalkyl mit 3, 4, 5 oder 6 C-Atomen, oder eine C_{b}H_{2b+1}-Gruppe, in der 1, 2, 3 oder 4 CH₂-Gruppen durch O, NH oder S ersetzt sein können;
b 1, 2, 3, 4, 5, 6, 7 oder 8;
- R(3): Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₈H₂ₐ₊₁)-Gruppe, in der 1, 2, 3 oder 4 CH₂-Gruppen durch O, NH oder S ersetzt sind;
a 1, 2, 3, 4, 5, 6, 7, 8, 9 oder 10;
oder
- R(3) und R(4): gemeinsam eine C_{b}H_{2b}-Gruppe, in der eine der CH₂-Gruppen durch ein Heteroatom ersetzt sein kann;
- b: 1, 2, 3, 4, 5, 6, 7 oder 8;
- R(4), R(5) und R(6): unabhängig voneinander Wasserstoff, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch Heteroatome, z.B. O, NH, S, ersetzt sind;
a 1, 2, 3, 4, 5, 6, 7, 8, 9 oder 10;
- E: Sauerstoff oder Schwefel;
- X: Sauerstoff oder Schwefel;
- Y: [CR(7)₂]₁₋₃, O, S oder NH;
R(7) Wasserstoff, Alkyl mit 1, 2, 3 oder 4 C-Atomen, F, Cl oder Br;
- Aryl: Phenyl, Thienyl, Furyl, Pyrrolyl, Thiazolyl, Naphthyl oder Pyridyl;
sowie ihre pharmazeutisch verträglichen Salze.

Besonders bevorzugt sind Verbindungen der Formel I, in denen bedeuten:
- R(1): Wasserstoff, Alkyl mit 1, 2, 3 oder 4 C-Atomen oder Cycloalkyl mit 3, 4, 5, 6 oder 7 C-Atomen;
- R(2): F, Cl, oder eine C_{b}H_{2b+1}-Gruppe, in der 1, 2, 3 oder 4 CH₂-Gruppen durch O, NH oder S ersetzt sein können;
b 1, 2, 3, 4, 5, 6, 7 oder 8;
- R(3): Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen, eine (CₐH₂ₐ₊₁)-Gruppe, in der 1, 2, 3 oder 4 CH₂-Gruppen durch O, NH oder S ersetzt sind;
a 1, 2, 3, 4, 5, 6, 7, 8, 9 oder 10;
oder
- R(3) und R(4): gemeinsam eine C_{b}H_{2b}-Gruppe, in der eine der CH₂-Gruppen durch O, NH oder S ersetzt sein kann;
b 1, 2, 3, 4, 5, 6, 7 oder 8;
- R(4), R(5) und R(6): Wasserstoff, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch O, NH oder S ersetzt sind;
a 1, 2, 3, 4, 5, 6, 7, 8, 9 oder 10;
- E: Sauerstoff oder Schwefel;
- X: Sauerstoff oder Schwefel;
- Y: [CR(7)₂]₁₋₃, O, S oder NH;
R(7) Wasserstoff, Alkyl mit 1, 2, 3 oder 4 C-Atomen, F, Cl oder Br;
- Aryl: Phenyl, Thienyl, Furyl, Pyrrolyl, Thiazolyl, Naphthyl oder Pyridyl;
sowie ihre pharmazeutisch verträglichen Salze

Eine bevorzugte Gruppe von Verbindungen der Formel I bilden zudem solche Verbindungen, in denen eine für R(2) stehende C_{b}H_{2b+1}-Gruppe, in der 1, 2, 3 oder 4 C-Atome durch Heteroatome 0, N oder S ersetzt sein können, für Alkyl mit 1, 2, 3, 4, 5 oder 6 C-Atomen, Alkoxy mit 1, 2, 3, 4, 5 oder 6 C-Atomen oder Mercaptoalkyl mit 1, 2, 3, 4, 5 oder 6 C-Atomen steht, sowie ihre pharmazeutisch verträglichen Salze.

Ganz besonders bevorzugt sind die Verbindungen der Formel I, in denen bedeuten:
- R(1): Wasserstoff, Alkyl mit 1, 2, 3 oder 4 C-Atomen, Cycloalkyl mit 3 oder 4 C-Atomen;
- R(2): Methoxy oder Ethoxy;
- R(3): Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 CH₂-Gruppen durch O, NH, S ersetzt sind;
a 1, 2, 3, 4, 5, 6, 7, 8, 9 oder 10;
oder
- R(3) und R(4): gemeinsam eine C_{b}H_{2b}-Gruppe, in der eine der CH₂-Gruppen durch O, NH oder S ersetzt sein kann;
b 1, 2, 3, 4, 5, 6, 7 oder 8;
- R(4), R(5) und R(6): unabhängig voneinander Wasserstoff, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch O, NH oder S ersetzt sind;
a 1, 2, 3, 4, 5, 6, 7, 8, 9 oder 10;
- E: Schwefel;
- X: Sauerstoff;
- Y: [CR(7)₂]₁₋₃, O, S oder NH;
R(7) Wasserstoff, Alkyl mit 1, 2, 3 oder 4 C-Atomen, F, Cl oder Br;
- Aryl: Phenyl, Thienyl, Furyl, Pyrrolyl, Thiazolyl, Naphthyl oder Pyridyl;
sowie ihre pharmazeutisch verträglichen Salze.

Ganz speziell bevorzugt sind Verbindungen der Formel I, in denen bedeuten:
- R(1): Wasserstoff, Alkyl mit 1, 2, 3 oder 4 C-Atomen oder Cycloalkyl mit 3 oder 4 C-Atomen;
- R(2): Methoxy oder Ethoxy;
- R(3): Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 CH₂-Gruppen durch O, NH oder S ersetzt sind;
a 1, 2, 3, 4, 5, 6, 7, 8, 9 oder 10;
oder
- R(3) und R(4): gemeinsam eine C_{b}H_{2b}-Gruppe, in der eine der CH₂-Gruppen durch O, S oder NH ersetzt sein kann;
b 1, 2, 3, 4, 5, 6, 7 oder 8;
- R(4), R(5) und R(6): unabhängig voneinander Wasserstoff, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch O, NH oder S ersetzt sind;
a 1, 2, 3, 4, 5, 6, 7, 8, 9 oder 10;
- E: Schwefel;
- X: Sauerstoff;
- Y: [CR(7)₂]₁₋₃;
R(7) Wasserstoff, Alkyl mit 1, 2, 3 oder 4 C-Atomen, F, Cl oder Br;
- Aryl: Phenyl, Thienyl, Furyl, Pyrrolyl, Thiazolyl, Naphthyl oder Pyridyl;
sowie ihre pharmazeutisch verträglichen Salze.

Insbesondere bevorzugt sind die Verbindungen der Formel I, in denen bedeuten
- R(1): Wasserstoff, Alkyl mit 1, 2, 3 oder 4 C-Atomen oder Cycloalkyl mit 3 oder 4 C-Atomen;
- R(2): Methoxy oder Ethoxy;
- R(3): Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 CH₂-Gruppen durch 0, S oder NH ersetzt sind;
a 1, 2, 3, 4, 5, 6, 7, 8, 9 oder 10;
oder
- R(3) und R(4): gemeinsam einen C_{b}H_{2b}-Gruppe, in der eine der CH₂-Gruppen durch O, S oder NH ersetzt sein kann;
b 1, 2, 3, 4, 5, 6, 7 oder 8;
- R(4), R(5) und R(6): unabhängig voneinander Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch O, NH oder S ersetzt sind;
a 1, 2, 3, 4, 5, 6, 7, 8, 9 oder 10;
- E: Schwefel;
- X: Sauerstoff;
- Y: CR(7)₂;
R(7) Wasserstoff oder Alkyl mit 1, 2, 3 oder 4 C-Atomen, F, Cl oder Br;
sowie ihre pharmazeutisch verträglichen Salze.

Außerdem sind insbesondere die Verbindungen bevorzugt, in denen bedeuten:
- R(1): Wasserstoff, Alkyl mit 1, 2, 3 oder 4 C-Atomen oder Cycloalkyl mit 3 oder 4 C-Atomen;
- R(2): Methoxy oder Ethoxy;
- R(3): Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 CH₂-Gruppen durch O, NH oder S ersetzt sind;
a 1, 2, 3, 4, 5, 6, 7, 8, 9 oder 10;
oder
- R(3) und R(4): gemeinsam eine C_{b}H_{2b}-Gruppe, in der eine der CH₂-Gruppen durch O, NH oder S ersetzt sein kann;
b 1, 2, 3, 4, 5, 6, 7 oder 8;
- R(4) und R(5): unabhängig voneinander Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch 0, NH oder S ersetzt sind;
a 1, 2, 3, 4, 5, 6, 7, 8, 9 oder 10;
- R(6): Aryl;
- E: Schwefel;
- X: Sauerstoff;
- Y: CR(7)₂;
R(7) Wasserstoff, Alkyl mit 1 oder 2 C-Atomen, F oder Cl;
- Aryl: Phenyl, Thienyl, Furyl, Pyrrolyl, Thiazolyl, Naphthyl oder Pyridyl;
sowie ihre pharmazeutisch verträglichen Salze.

Die Verbindungen I der vorliegenden Erfindung sind wertvolle Arzneimittel zur Behandlung von Herzrhythmusstörungen unterschiedlichster Genese und zur Verhinderung des arrhythmisch bedingten, plötzlichen Herztods und können daher als Antiarrhythmika Verwendung finden. Beispiele arrhythmischer Störungen des Herzens sind supraventrikuläre Rhythmusstörungen wie etwa Vorhof-Tachykardien, Vorhof-Flattern oder paroxysmale supraventrikuläre Rhythmusstörungen, oder ventrikuläre Rhythmusstörungen, wie ventrikuläre Extrasystolen, insbesondere aber lebensbedrohende ventrikuläre Tachykardien oder das besonders gefährliche Kammerflimmern. Sie eignen sich insbesondere für solche Fälle, in denen Arrhythmien Folge einer Verengung eines Koronargefäßes sind, wie sie beispielsweise bei Angina Pectoris oder während eines akuten Herzinfarkts oder als chronische Folge eines Herzinfarkts auftreten. Sie sind daher insbesondere bei Postinfarktpatienten zur Verhinderung des plötzlichen Herztods geeignet. Weitere Krankheitsbilder, wo derartige Rhythmusstörungen und/oder der plötzliche, arrhythmisch bedingte Herztod eine Rolle spielen, sind beispielsweise die Herzinsuffizienz oder die Herzhypertrophie als Folge eines chronisch erhöhten Blutdrucks.

Darüber hinaus können die Verbindungen I eine verminderte Kontraktilität des Herzens positiv beeinflussen. Hierbei kann es sich um ein krankheitsbedingtes Nachlassen der Herzkontraktilität handeln, beispielsweise bei Herzinsuffizienz aber auch um akute Fälle wie Herzversagen bei Schockeinwirkungen. Ebenso kann bei einer Herztransplantation das Herz nach erfolgter Operation seine Leistungsfähigkeit rascher und zuverlässiger wieder aufnehmen. Gleiches gilt für Operationen am Herz, die eine vorübergehende Stillegung der Herzaktivität durch kardioplegische Lösungen erforderlich machen, wobei die Verbindungen sowohl für den Schutz der Organe beispielsweise bei Behandlung mit oder deren Lagerung in physiologischen Badflüssigkeiten, wie auch bei der Überführung in den Empfängerorganismus verwendet werden können.

Die Erfindung betrifft weiterhin ein Verfahren zur Herstellung der Verbindungen I, dadurch gekennzeichnet, daß man
(a) ein Sulfonamid der Formel II 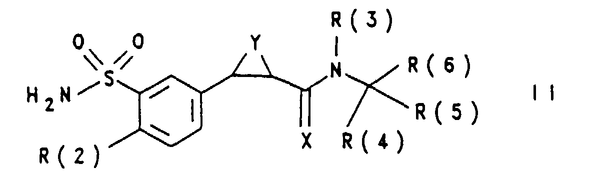 oder dessen Salz der Formel III 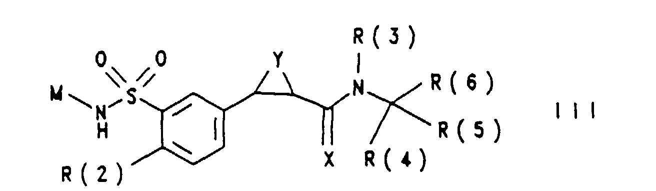 mit einem R(1)-substituierten Isocyanat der Formel IV

   R(1) - N = C = O IV

   zu einem substituierten Benzolsulfonylharnstoff I a umsetzt.
   Als Kationen M in den Salzen der Formel III kommen Alkali- und Erdalkaliionen in Betracht. Äquivalent zu den R(1)-substituierten Isocyanaten IV kann man R(1)-substituierte Carbamidsäureester, R(1)-substituierte Carbamidsäure-halogenide oder R(1)-substituierte Harnstoffe einsetzen.
(b) Ein Benzolsulfonylharnstoff I a 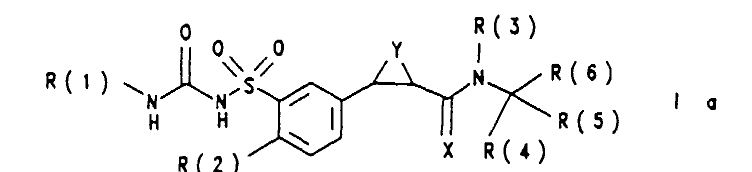 läßt sich aus einem aromatischen Benzolsulfonamid II oder dessen Salz III mit einem R(1)-substituierten Trichloracetamid der Formel V

   Cl₃C-(CO)-NHR(1) V

   in Gegenwart einer Base in einem inerten Lösungsmittel nach Synthesis 1987, 734 - 735 bei Temperaturen von 25°C bis 150°C darstellen.
   Als Basen eignen sich zum Beispiel Alkalimetall- oder Erdalkalimetallhydroxide, oder auch -alkoholate, wie Natriumhydroxid, Kaliumhydroxid, Calciumhydroxid, Natriummethylat, Natriumethanolat, Kaliummethylat oder Kaliumethanolat. Als inerte Lösungsmittel eignen sich Ether wie Tetrahydrofuran, Dioxan, Ethylenglykoldimethylether (Diglyme), Nitrile wie Acetonitril, Amide wie Dimethylformamid (DMF) oder N-Methylpyrrolidon (NMP), Phosphorsäurehexamethyltriamid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander.
(c) Ein Benzolsulfonylthioharnstoff I b 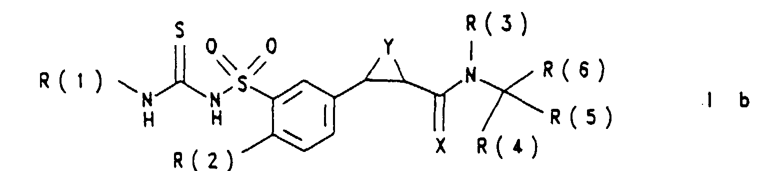 wird aus einem Benzolsulfonamid II oder dessen Salz III und einem R(1)-substituierten Thioisocyanat VI

   R(1) - N = C = S VI

   dargestellt.
(d) Ein substituierter Benzolsulfonylharnstoff der Formel I a kann durch Umwandlungsreaktion von einem Benzolsulfonylthioharnstoff der Struktur I b dargestellt werden. Der Ersatz des Schwefelatoms durch ein Sauerstoffatom in den entsprechend substituierten Benzolsulfonylthioharnstoffen I b kann man beispielsweise mit Hilfe von Oxiden oder Salzen von Schwermetallen oder auch durch Anwendung von Oxidationsmitteln wie Wasserstoffperoxid, Natriumperoxid oder Salpetersäure ausgeführt werden. Ein Thioharnstoff kann auch durch Behandlung mit Phosgen oder Phosphorpentachlorid entschwefelt werden. Als Zwischenverbindungen werden Chlorameisensäureamidine bzw. Carbodiimide erhalten, die zum Beispiel durch Verseifen oder Anlagerung von Wasser in die entsprechenden substituierten Benzolsulfonylharnstoffe I a überführt werden. Isothioharnstoffe verhalten sich bei der Entschwefelung wie Thioharnstoffe und können demzufolge ebenso als Ausgangsstoffe für diese Reaktionen dienen.
(e) Ein Benzolsulfonylharnstoff I läßt sich durch Umsetzung eines Amins der Formel R(1)-NH₂ mit einem Benzolsulfonylisocyanat der Formel VII 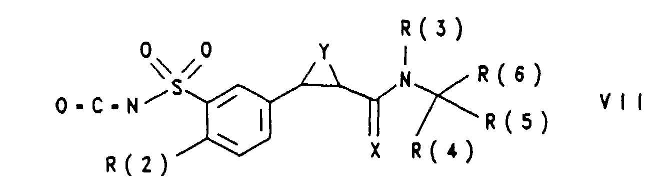 herstellen. Ebenso kann ein Amin R(1)-NH₂ mit einem Benzolsulfonylcarbamidsäureester, -carbamidsäurehalogenid oder Benzolsulfonylharnstoff I a [mit R(1) = H] zu einer Verbindung I umgesetzt werden.
(f) Ein Benzolsulfonylthioharnstoff I b läßt sich durch Umsetzung eines Amins der Formel R(1)-NH₂ mit einem Benzolsulfonylisothiocyanat der Formel VIII 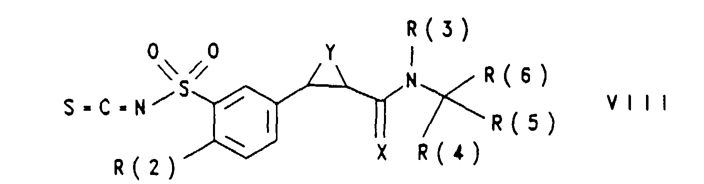 herstellen. Die Herstellung der Sulfonylisothiocyanate erfolgte durch Umsetzung der entsprechenden Sulfonsäureamide mit äquimolaren Mengen Alkalihydroxiden und Schwefelkohlenstoff in einem organischen Lösungsmittel wie z.B. DMF, DMSO, N-Methylpyrrolidon. Das so erhaltene Di-alkalimetall-Salz der Sulfonyldithiocarbaminsäure wird in einem inertem Lösungsmittel mit einem leichten Überschuß von Phosgen, bzw. Ersatzstoff desgleichen wie z.B. Triphosgen, Chlorameisensäureestern (2 Äquivalente) oder Thionylchlorid umgesetzt. Die so erhaltene Lösung des Sulfonylisothiocyanats kann direkt mit den entsprechenden Aminen oder Ammoniak umgesetzt werden.
(g) Ein Benzolsulfonylharnstoff I a kann aus einem Benzolsulfonylharnstoff der Formel IX a und R(3)R(4)NH mittels wasserentziehender Mittel oder Aktivierung mittels Carbonsäurehalogenide bzw. Bildung gemischter Anhydride dargestellt werden: 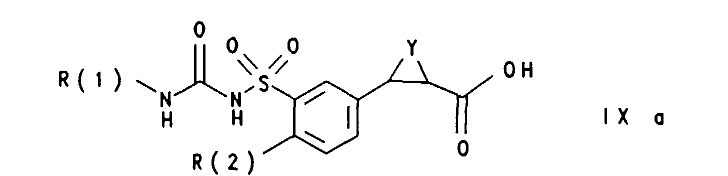 Als wasserentziehende Mittel können alle zur Herstellung von Amidbindungen geeignete Verbindungen eingesetzt werden, wie z.B. Dicyclohexylcarbodiimid, Carbonylbisimidazol, Propanphosphosphorsäureanhydrid. Als Lösungsmittel finden inerte nicht protische Lösungsmittel wie THF, DMF, Diethylether, Dichlormethan, sowie Gemische dieser Lösungsmittel Anwendung.
(h) Ein Benzolsulfonylthioharnstoff I b kann aus einem Benzolsulfonylthioharnstoff der Formel IX b und R(3)R(4)NH mittels wasserentziehender Mittel oder Aktivierung mittels Carbonsäurehalogenide bzw. Bildung gemischter Anhydride dargestellt werden:

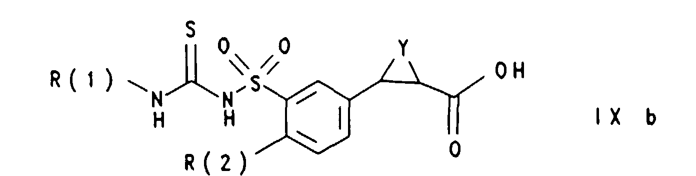

Als wasserentziehende Mittel können alle zur Herstellung von Amidbindungen geeignete Verbindungen eingesetzt werden, wie z.B. Dicyclohexylcarbodiimid, Carbonylbisimidazol, Propanphosphosphorsäureanhydrid. Als Lösungsmittel finden inerte nicht protische Lösungsmittel wie THF, DMF, Diethylether, Dichlormethan, sowie Gemische dieser Lösungsmittel Anwendung.

Die Verbindungen I sowie deren physiologisch unbedenklichen Salze sind wertvolle Therapeutika, die sich nicht nur als Antiarrhythmika, sondern auch bei der Prophylaxe bei Störungen des kardiovaskulären Systems, Herzinsuffizienz, Herztranplantation oder cerebralen Gefäßerkrankungen an Menschen oder Säugetieren (zum Beispiel Affen, Hunde, Mäuse, Ratten, Kaninchen, Meerschweinchen und Katzen) eignen.

Unter physiologisch unbedenklichen Salzen der Verbindungen I versteht man nach Remmington's Pharmaceutical Science, 17. Auflage, 1985, Seiten 14-18 Verbindungen der Formel X, 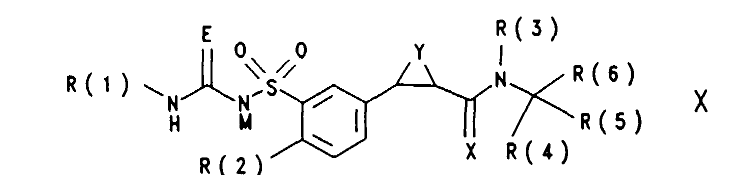 die sich aus nicht toxischen organischen und anorganischen Basen und Benzolsulfonylharnstoffen I darstellen lassen.

Bevorzugt werden hierbei Salze in denen M(1) in der Formel X Natrium-, Kalium-, Rubidium-, Calcium-, Magnesiumionen sind, sowie die Säureadditionsprodukte basischer Aminosäuren, wie zum Beispiel Lysin oder Arginin.

Die Ausgangsverbindungen für die erwähnten Syntheseverfahren der Benzolsulfonylharnstoffe I werden nach bekannten Methoden hergestellt, wie sie in der Literatur (zum Beispiel in den Standardwerken wie Houben-Weyl, Methoden der Organischen Chemie, Georg Thieme Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

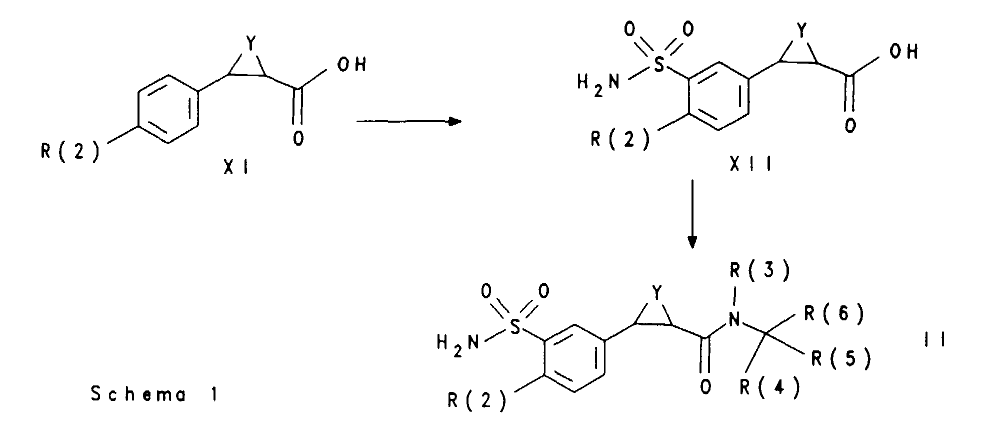

So kann man geeignet substituierte Carbonsäuren der Formel XI nach Schema 1 einer Halogensulfonierung unterwerfen und das durch anschließende Ammonolyse erhaltene Sulfonamid XII mit entsprechenden Aminen R(3)R(4)NH nach Aktivierung der Carbonsäuregruppe zum Carbonsäureamid der Formel II umsetzen.
Als Aktivierungsmethoden eignen sich die Herstellung des Carbonsäurechlorids oder gemischter Carbonsäureanhydride mit Ameisensäurehalogeniden. Desweiteren können die zur Amidbindungsherstellung bekannten Reagenzien wie zum Beispiel Carbonylbisimidazol, Dicyclohexylcarbodiimid und Propanphosphorsäureanhydrid angewandt werden.

Die in Schema 1 als Zwischenprodukte erhaltenen Sulfonamide XII können mit entsprechenden Isocyanaten der Formel R(1)-N=C=E zu den Benzolsulfonylharnstoffcarbonsäuren der Formel IX nach Schema 2 umgesetzt werden.

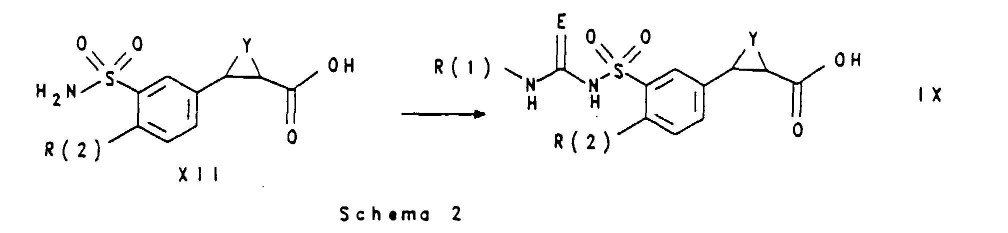

Die Verbindungen I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Isomere, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen eignen sich zum Beispiel optisch aktive Säuren, wie die R- bzw. R,R- und S- bzw. S,S-Formen von Weinsäure, Dibenzoylweinsäure, Diacetylweinsäure, Camphersulfonsäuren, Mandelsäuren, Äpfelsäure oder Milchsäure. Carbinole können ferner mit Hilfe chiraler Acylierungsreagenzien, zum Beispiel R- oder S-a-Methylbenzylisocyanat amidiert und dann getrennt werden. Die verschiedenen Formen der Diastereomeren können in an sich bekannter Weise, zum Beispiel durch fraktionierte Kristallisation, getrennt und die Enantiomeren der Formel I können in bekannter Weise aus den Diastereomeren in Freiheit gesetzt werden.
Enantiomerentrennungen gelingen ferner durch Chromatographie an optischaktiven Trägermaterialien.

Die erfindungsgemäßen Verbindungen I und ihre physiologisch unbedenklichen Salze können zur Herstellung pharmazeutischer Zubereitungen verwendet werden. Hierbei können sie zusammen mit mindestens einem festen oder flüssigen Träger oder Hilfsstoff allein oder in Kombination mit anderen Herz-Kreislauf-aktiven Arzneimitteln, wie etwa Calcium-Antagonisten, NO-Donatoren oder ACE-Hemmern in eine geeignete Dosierungsform gebracht werden. Diese Zubereitungen können als Arzneimittel in der Human- oder Veterinärmedizin verwendet werden. Als Trägerstoffe kommen organische oder anorganische Substanzen in Frage, die sich für die enterale (zum Beispiel orale), parenterale, wie zum Beispiel die intravenöse Applikation, oder topische Anwendungen eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Lösungen, vorzugsweise ölige oder wäßrige Lösungen, ferner Suspensionen, Emulsionen oder Implantate, für die topische Anwendung Salben, Cremes, Pasten, Lotionen, Gele, Sprays, Schäume, Aerosole, Lösungen (zum Beispiel in Alkoholen, wie Ethanol oder Isopropanol, Acetonitril, 1,2-Propandiol oder deren Gemische untereinander oder mit Wasser) oder Puder. Die neuen Verbindungen können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht, die Stabilisierungs- und/oder Netzmittel, Emulgatoren, Salze und/oder Hilfsstoffe wie Gleit- und Konservierungsmittel, Salze zur Beeinflussung des osmotischen Druckes, Puffersubstanzen, Farb- und Geschmacks- und/oder Aromastoffe enthalten. Sie können, falls erwünscht, auch einen oder mehrere weitere Wirkstoffe enthalten, zum Beispiel ein oder mehrere Vitamine.

Die Dosierungen, die zur Behandlung von Herzrhythmusstörungen mit den Verbindungen I notwendig sind, hängen davon ab, ob akut oder prophylaktisch therapiert wird. Normalerweise kommt man mit einem Dosisbereich von etwa mindestens 0.1 mg, vorzugsweise mindestens 1 mg, bis höchstens 100 mg, vorzugsweise bis höchstens 10 mg pro kg und Tag aus, wenn Prophylaxe betrieben wird. Bevorzugt ist ein Dosisbereich von 1 bis 10 mg pro kg und Tag, bezogen auf einen Erwachsenen von 75 kg Durchschnittsgewicht. Die Dosis kann dabei als orale oder parenterale Einzeldosis oder in bis zu vier Einzeldosen aufgeteilt werden. Werden akute Fälle von Herzrhythmusstörungen behandelt, beispielsweise auf einer Intensivstation, kann die parenterale Verabreichung vorteilhaft sein. Ein bevorzugte Dosisbereich in kritischen Situationen kann dann 10 bis 100 mg betragen und beispielsweise als intravenöse Dauerinfusion verabreicht werden.

Erfindungsgemäß können außer den in den Ausführungsbeispielen beschriebenen Verbindungen auch die in der folgenden Tabelle zusammengestellten Verbindungen I erhalten werden:
(1) 3-Sulfonylamino-N-(methylaminocarbonyl)-4-methoxyphenylcyclopropancarbonsäure-N-methylamid,
(2) 3-Sulfonylamino-N-(methylaminocarbonyl)-4-methoxyphenylcyclopropancarbonsäure-N-ethylamid
(3) 3-Sulfonylamino-N-(methylaminocarbonyl)-4-methoxyphenylcyclopropancarbonsäure-N-1-propylamid
(4) 3-Sulfonylamino-N-(methylaminocarbonyl)-4-methoxyphenylcyclopropancarbonsäure-N-2-propylamid
(5) 3-Sulfonylamino-N-(methylaminocarbonyl)-4-methoxyphenylcyclopropancarbonsäure-N-1-butylamid
(6) 3-Sulfonylamino-N-(methylaminocarbonyl)-4-methoxyphenylcyclopropancarbonsäure-N-2-butylamid
(7) 3-Sulfonylamino-N-(methylaminocarbonyl)-4-methoxyphenylcyclopropancarbonsäure-N-1-pentylamid
(8) 3-Sulfonylamino-N-(methylaminocarbonyl)-4-methoxyphenylcyclopropancarbonsäure-N-2-pentylamid
(9) 3-Sulfonylamino-N-(methylaminocarbonyl)-4-methoxyphenylcyclopropancarbonsäure-N-3-pentylamid
(10) 3-Sulfonylamino-N-(methylaminocarbonyl)-4-methoxyphenylcyclopropancarbonsäure-N-1-butyl-2-methylamid
(11) 3-Sulfonylamino-N-(methylaminocarbonyl)-4-methoxyphenylcyclopropancarbonsäure-N-1-butyl-3-methylamid
(12) 3-Sulfonylamino-N-(methylaminocarbonyl)-4-methoxyphenylcyclopropancarbonsäure-N-1-hexylamid
(13) 3-Sulfonylamino-N-(methylaminocarbonyl)-4-methoxyphenylcyclopropancarbonsäure-N-2-hexylamid
(14) 3-Sulfonylamino-N-(methylaminocarbonyl)-4-methoxyphenylcyclopropancarbonsäure-N-3-hexylamid
(15) 3-Sulfonylamino-N-(methylaminocarbonyl)-4-methoxyphenylcyclopropancarbonsäure-N-1-heptylamid
(16) 3-Sulfonylamino-N-(methylaminocarbonyl)-4-methoxyphenylcyclopropancarbonsäure-N-2-heptylamid
(17) 3-Sulfonylamino-N-(methylaminocarbonyl)-4-methoxyphenylcyclopropancarbonsäure-N-3-heptylamid
(18) 3-Sulfonylamino-N-(methylaminocarbonyl)-4-methoxyphenylcyclopropancarbonsäure-N-1-octylamid
(19) 3-Sulfonylamino-N-(methylaminocarbonyl)-4-methoxyphenylcyclopropancarbonsäure-N-2-octylamid
(20) 3-Sulfonylamino-N-(methylaminocarbonyl)-4-methoxyphenylcyclopropancarbonsäure-N-1-adamantylamid
(21) 3-Sulfonylamino-N-(methylaminocarbonyl)-4-methoxyphenylcyclopropancarbonsäure-N-2-adamantylamid
(22) 3-Sulfonylamino-N-(methylaminocarbonyl)-4-methoxyphenylcyclopropancarbonsäure-N-di-methyl-amid
(23) 3-Sulfonylamino-N-(methylaminocarbonyl)-4-methoxyphenylcyclopropancarbonsäure-N-methyl-N'-ethylamid
(24) 3-Sulfonylamino-N-(methylaminocarbonyl)-4-methoxyphenylcyclopropancarbonsäure-N-methyl-N'-1-propylamid
(25) 3-Sulfonylamino-N-(methylaminocarbonyl)-4-methoxyphenylcyclopropancarbonsäure-N-methyl-N'-2-propylamid
(26) 3-Sulfonylamino-N-(methylaminocarbonyl)-4-methoxyphenylcyclopropancarbonsäure-N-methyl-N'-1-butylamid
(27) 3-Sulfonylamino-N-(methylaminocarbonyl)-4-methoxyphenylcyclopropancarbonsäure-N-methyl-N'-2-butylamid
(28) 3-Sulfonylamino-N-(methylaminocarbonyl)-4-methoxyphenylcyclopropancarbonsäure-N-di-ethylamid
(29) 3-Sulfonylamino-N-(methylaminocarbonyl)-4-methoxyphenylcyclopropancarbonsäure-N-ethyl-N'-1-propylamid
(30) 3-Sulfonylamino-N-(methylaminocarbonyl)-4-methoxyphenylcyclopropancarbonsäure-N-ethyl-N'-2-propylamid
(31) 3-Sulfonylamino-N-(methylaminocarbonyl)-4-methoxyphenylcyclopropancarbonsäure-pyrrolidinylamid
(32) 3-Sulfonylamino-N-(methylaminocarbonyl)-4-methoxyphenylcyclopropancarbonsäure-piperidylamid
(33) 3-Sulfonylamino-N-(methylaminocarbonyl)-4-methoxyphenylcyclopropancarbonsäure-morpholinoamid
(34) 3-Sulfonylamino-N-(methylaminocarbonyl)-4-methoxyphenylcyclopropancarbonsäure-(N-methyl-piperazinyl)amid
(35) 3-Sulfonylamino-N-(methylaminocarbonyl)-4-methoxyphenylcyclopropancarbonsäure-4-thio-morpholinylamid
(36) 3-Sulfonylamino-N-(methylaminothiocarbonyl)-4-methoxyphenylcyclopropancarbonsäure-N-methylamid,
(37) 3-Sulfonylamino-N-(methylaminothiocarbonyl)-4-methoxyphenylcyclopropancarbonsäure-N-ethylamid
(38) 3-Sulfonylamino-N-(methylaminothiocarbonyl)-4-methoxyphenylcyclopropancarbonsäure-N-1-propylamid
(39) 3-Sulfonylamino-N-(methylaminothiocarbonyl)-4-methoxyphenylcyclopropancarbonsäure-N-2-propylamid
(40) 3-Sulfonylamino-N-(methylaminothiocarbonyl)-4-methoxyphenylcyclopropancarbonsäure-N-1-butylamid
(41) 3-Sulfonylamino-N-(methylaminothiocarbonyl)-4-methoxyphenylcyclopropancarbonsäure-N-2-butylamid
(42) 3-Sulfonylamino-N-(methylaminothiocarbonyl)-4-methoxyphenylcyclopropancarbonsäure-N-1-pentylamid
(43) 3-Sulfonylamino-N-(methylaminothiocarbonyl)-4-methoxyphenylcyclopropancarbonsäure-N-2-pentylamid
(44) 3-Sulfonylamino-N-(methylaminothiocarbonyl)-4-methoxyphenylcyclopropancarbonsäure-N-3-pentylamid
(45) 3-Sulfonylamino-N-(methylaminothiocarbonyl)-4-methoxyphenylcyclopropancarbonsäure-N-1-butyl-2-methylamid
(46) 3-Sulfonylamino-N-(methylaminothiocarbonyl)-4-methoxyphenylcyclopropancarbonsäure-N-1-butyl-3-methylamid
(47) 3-Sulfonylamino-N-(methylaminothiocarbonyl)-4-methoxyphenylcyclopropancarbonsäure-N-1-hexylamid
(48) 3-Sulfonylamino-N-(methylaminothiocarbonyl)-4-methoxyphenylcyclopropancarbonsäure-N-2-hexylamid
(49) 3-Sulfonylamino-N-(methylaminothiocarbonyl)-4-methoxyphenylcyclopropancarbonsäure-N-3-hexylamid
(50) 3-Sulfonylamino-N-(methylaminothiocarbonyl)-4-methoxyphenylcyclopropancarbonsäure-N-1-heptylamid
(51) 3-Sulfonylamino-N-(methylaminothiocarbonyl)-4-methoxyphenylcyclopropancarbonsäure-N-2-heptylamid
(52) 3-Sulfonylamino-N-(methylaminothiocarbonyl)-4-methoxyphenylcyclopropancarbonsäure-N-3-heptylamid
(53) 3-Sulfonylamino-N-(methylaminothiocarbonyl)-4-methoxyphenylcyclopropancarbonsäure-N-1-octylamid
(54) 3-Sulfonylamino-N-(methylaminothiocarbonyl)-4-methoxyphenylcyclopropancarbonsäure-N-2-octylamid
(55) 3-Sulfonylamino-N-(methylaminothiocarbonyl)-4-methoxyphenylcyclopropancarbonsäure-N-1-adamantylamid
(56) 3-Sulfonylamino-N-(methylaminothiocarbonyl)-4-methoxyphenylcyclopropancarbonsäure-N-2-adamantylamid
(57) 3-Sulfonylamino-N-(methylaminothiocarbonyl)-4-methoxyphenylcyclopropancarbonsäure-N-di-methyl-amid
(58) 3-Sulfonylamino-N-(methylaminothiocarbonyl)-4-methoxyphenylcyclopropancarbonsäure-N-methyl-N'-ethylamid
(59) 3-Sulfonylamino-N-(methylaminothiocarbonyl)-4-methoxyphenylcyclopropancarbonsäure-N-methyl-N'-1-propylamid
(60) 3-Sulfonylamino-N-(methylaminothiocarbonyl)-4-methoxyphenylcyclopropancarbonsäure-N-methyl-N'-2-propylamid
(61) 3-Sulfonylamino-N-(methylaminothiocarbonyl)-4-methoxyphenylcyclopropancarbonsäure-N-methyl-N'-1-butylamid
(62) 3-Sulfonylamino-N-(methylaminothiocarbonyl)-4-methoxyphenylcyclopropancarbonsäure-N-methyl-N'-2-butylamid
(63) 3-Sulfonylamino-N-(methylaminothiocarbonyl)-4-methoxyphenylcyclopropancarbonsäure-N-di-ethylamid
(64) 3-Sulfonylamino-N-(methylaminothiocarbonyl)-4-methoxyphenylcyclopropancarbonsäure-N-ethyl-N'-1-propylamid
(65) 3-Sulfonylamino-N-(methylaminothiocarbonyl)-4-methoxyphenylcyclopropancarbonsäure-N-ethyl-N'-2-propylamid
(66) 3-Sulfonylamino-N-(methylaminothiocarbonyl)-4-methoxyphenylcyclopropancarbonsäure-pyrrolidinylamid
(67) 3-Sulfonylamino-N-(methylaminothiocarbonyl)-4-methoxyphenylcyclopropancarbonsäure-piperidylamid
(68) 3-Sulfonylamino-N-(methylaminothiocarbonyl)-4-methoxyphenylcyclopropancarbonsäure-morpholinoamid
(69) 3-Sulfonylamino-N-(methylaminothiocarbonyl)-4-methoxyphenylcyclopropancarbonsäure-(N-methyl-piperazinyl)amid
(70) 3-Sulfonylamino-N-(methylaminothiocarbonyl)-4-methoxyphenylcyclopropancarbonsäure-4-thio-morpholinylamid
(71) 3-Sulfonylamino-N-(methylaminothiocarbonyl)-4-methylphenylcyclopropancarbonsäure-N-methylamid,
(72) 3-Sulfonylamino-N-(methylaminothiocarbonyl)-4-methylphenylcyclopropancarbonsäure-N-ethylamid
(73) 3-Sulfonylamino-N-(methylaminothiocarbonyl)-4-methylphenylcyclopropancarbonsäure-N-1-propylamid
(74) 3-Sulfonylamino-N-(methylaminothiocarbonyl)-4-methylphenylcyclopropancarbonsäure-N-2-propylamid
(75) 3-Sulfonylamino-N-(methylaminothiocarbonyl)-4-methylphenylcyclopropancarbonsäure-N-1-butylamid
(76) 3-Sulfonylamino-N-(methylaminothiocarbonyl)-4-methylphenylcyclopropancarbonsäure-N-2-butylamid
(77) 3-Sulfonylamino-N-(methylaminothiocarbonyl)-4-methylphenylcyclopropancarbonsäure-N-1-pentylamid
(78) 3-Sulfonylamino-N-(methylaminothiocarbonyl)-4-methylphenylcyclopropancarbonsäure-N-2-pentylamid
(79) 3-Sulfonylamino-N-(methylaminothiocarbonyl)-4-methylphenylcyclopropancarbonsäure-N-3-pentylamid
(80) 3-Sulfonylamino-N-(methylaminothiocarbonyl)-4-methylphenylcyclopropancarbonsäure-N-1-butyl-2-methylamid
(81) 3-Sulfonylamino-N-(methylaminothiocarbonyl)-4-methylphenylcyclopropancarbonsäure-N-1-butyl-3-methylamid
(82) 3-Sulfonylamino-N-(methylaminothiocarbonyl)-4-methylphenylcyclopropancarbonsäure-N-1-hexylamid
(83) 3-Sulfonylamino-N-(methylaminothiocarbonyl)-4-methylphenylcyclopropancarbonsäure-N-2-hexylamid
(84) 3-Sulfonylamino-N-(methylaminothiocarbonyl)-4-methylphenylcyclopropancarbonsäure-N-3-hexylamid
(85) 3-Sulfonylamino-N-(methylaminothiocarbonyl)-4-methylphenylcyclopropancarbonsäure-N-1-heptylamid
(86) 3-Sulfonylamino-N-(methylaminothiocarbonyl)-4-methylphenylcyclopropancarbonsäure-N-2-heptylamid
(87) 3-Sulfonylamino-N-(methylaminothiocarbonyl)-4-methylphenylcyclopropancarbonsäure-N-3-heptylamid
(88) 3-Sulfonylamino-N-(methylaminothiocarbonyl)-4-methylphenylcyclopropancarbonsäure-N-1-octylamid
(89) 3-Sulfonylamino-N-(methylaminothiocarbonyl)-4-methylphenylcyclopropancarbonsäure-N-2-octylamid
(90) 3-Sulfonylamino-N-(methylaminothiocarbonyl)-4-methylphenylcyclopropancarbonsäure-N-1-adamantylamid
(91) 3-Sulfonylamino-N-(methylaminothiocarbonyl)-4-methylphenylcyclopropancarbonsäure-N-2-adamantylamid

### Herstellung der Ausgangsmaterialien

### Herstellung von 2-(3-Sulfonylamino-4-methoxy-phenyl)cyclopropancarbonsäuren

Die 4-substituierten Phenylcarbonsäureester werden unter Rühren portionsweise zu einem Überschuß an Chlorsulfonsäure gegeben. Man rührt 30 min bei Raumtemperatur gießt anschließend auf Eis und saugt das entstandene Sulfonsäurechlorid ab. Dieses wird in Ammoniaklösung gelöst, 30 min bei Raumtemperatur gerührt und die Lösung mit 2 N Salzsäure neutralisiert. Das erhaltene Produkt wird abgesaugt. Die erhaltenen Ester werden mit einem Überschuß an Lithiumhydroxid in einer THF/Wasser-Mischung über Nacht bei Raumtemperatur gerührt. Durch Ansäuern mit 2 N HCI und Extrahieren mit Essigester erhält man die gewünschten Säuren.
Nach dieser Methode hergestellt:
2-(3-Sulfonylamino-4-methoxy-phenyl)cyclopropan-carbonsäureethylester. Smp. 154°C 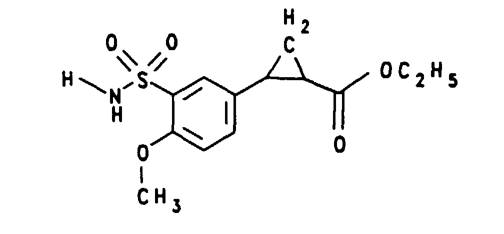 2-(3-Sulfonylamino-4-methoxy-phenyl)cyclopropan-carbonsäure. Smp. 160°C.

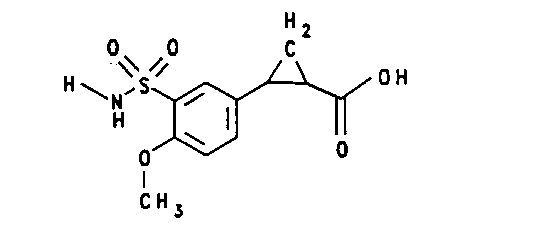

### Beispiel 1

2-[3-Sulfonylamino-N-(methylaminothiocarbonyl)-4-methoxyphenyl]-cyclopropancarbonsäure-N-1-phenyl-1-butyl-amid 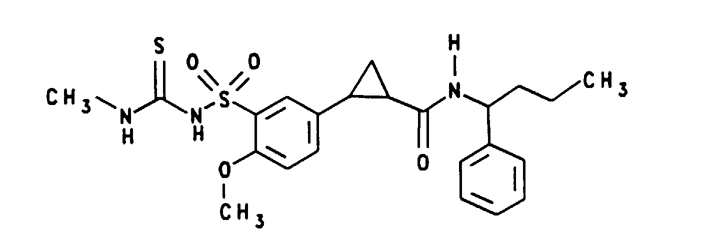 250 mg 2-(3-Sulfonylamino-4-methoxyphenyl)-cyclopropancarbonsäure-N-1-phenyl-1-butyl-amid werden in 3 ml DMF gelöst, mit 37 mg NaOH versetzt und 30 min bei 40°C gerührt. Anschließend gibt man 54 mg Methylisothiocyanat zu und rührt weitere 2 h bei 70°C. Nach Abkühlen und Neutralisieren mit 2 N Salzsäure saugt man das Produkt ab und trocknet.
Smp.110°C.

### Pharmakologische Daten:

Mit den folgenden Modellen können die therapeutischen Eigenschaften der Verbindungen 1 nahegelegt werden:

### (1) Aktionspotentialdauer am Papillarmuskel des Meerschweinchens:

### (a) Einleitung

ATP-Mangelzustände wie sie während einer Ischämie in der Herzmuskelzelle beobachtet werden führen zu einer Verkürzung der Aktionspotentialdauer. Sie gelten als einer der Ursachen für sogenannte Reentry-Arrhythmien die den plötzlichen Herztod verursachen können. Die Öffnung von ATP-sensitiven K-Kanälen durch das Absinken von ATP gilt hierfür als ursächlich.

### (b) Methode

Zur Messung des Aktionspotentials wird eine Standard-Mikroelektroden-Technik eingesetzt. Hierfür werden Meerschweinchen beiderlei Geschlechts durch Schlag auf den Kopf getötet, die Herzen entnommen, die Papillarmuskeln herausgetrennt und in einem Organbad aufgehängt. Das Organbad wird mit Ringerlösung (0.9% NaCl, 0.048% KCl, 0.024% CaCl₂, 0.02% NaHCO₃ und 0.1 % Glucose) durchspült und mit einer Mischung aus 95% Sauerstoff und 5% Kohlendioxid bei einer Temperatur von 36°C begast. Der Muskel wird über eine Elektrode mit Rechteck-Impulsen von 1 V und 1 ms Dauer und einer Frequenz von 2 Hz angeregt. Das Aktionspotential wird durch eine intrazellulär eingestochene Glas-Mikroelektrode, die mit 3 mMol KCl-Lösung gefüllt ist, abgeleitet und registriert. Die zu prüfenden Substanzen wurden der Ringerlösung in einer Konzentration von 2.2·10⁻⁵ Mol pro Liter zugesetzt. Das Aktionspotential wird mit einem Amplifier von Hugo Sachs verstärkt und am Oszilloskop dargestellt. Die Dauer des Aktionspotentials wird bei einem Repolarisierungsgrad von 95% (APD95) bestimmt.
Aktionspotentialverkürzungen werden entweder durch Zugabe einer 1 µM starken Lösung des Kaliumkanalöffners Hoe 234 (J. Kaiser, H. Gögelein, Naunyn-Schmiedebergs Arch. Pharm. 1991, 343, R 59) oder durch Zugabe von 2-Desoxyglucose hervorgerufen. Der aktionspotentialverkürzende Effekt dieser Substanzen wurde durch die gleichzeitige Gabe der Testsubstanzen verhindert oder verringert. Testsubstanzen wurden als Stammlösungen in Propandiol der Badlösung zugesetzt. Die angegebenen Werte beziehen sich auf Messungen 30 min nach der Zugabe. Glibenclamid diente in diesen Messungen als Standard. Die Testkonzentration beträgt in allen Fällen 2 x 10⁻⁶ M.

### (c) Resultate:

Folgende Werte wurden gemessen:.

| Beispiel Nr. | APD95-Anfang [ms] | APD95-30 min [ms] |
|---|---|---|
| 1 | 160 ± 8 | 122 ± 16 |

## Patentansprüche

1. Substituierte Benzolsulfonylharnstoffe und -thioharnstoffe der Formel I 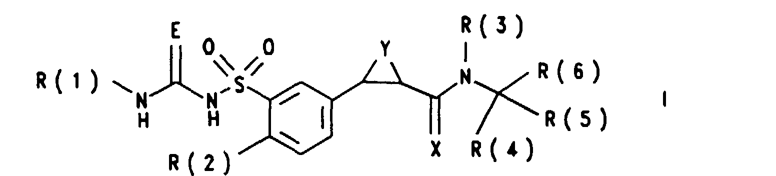 worin bedeuten:
R(1) Wasserstoff, Alkyl mit 1, 2, 3, 4, 5, 6 oder 7 C-Atomen, Cycloalkyl mit 3, 4, 5, 6 oder 7 C-Atomen;
R(2) Wasserstoff, F, Cl, Cycloalkyl mit 3, 4, 5 oder 6 C-Atomen oder eine C_{b}H_{2b+1}-Gruppe, in der 1, 2, 3 oder 4 C-Atome durch Heteroatome O, N oder S ersetzt sein können;
b 1, 2, 3, 4, 5, 6, 7 oder 8;
R(3) H, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch Heteroatome, z.B. O, NH, S, ersetzt sind;
a 1, 2, 3, 4, 5, 6, 7, 8, 9 oder 10;
R(4) H, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch Heteroatome, z.B. O, NH oder S, ersetzt sind;
a 1, 2, 3, 4, 5, 6, 7, 8, 9 oder 10;
oder
R(3) und R(4) gemeinsam eine C_{b}H_{2b}-Gruppe, in der eine oder mehrere der CH₂-Gruppen durch Heteroatome, z. B. durch O, S oder NH, ersetzt sein können;
b 1, 2, 3, 4, 5, 6, 7 oder 8;
R(5) H, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe in der 1, 2, 3 oder 4 C-Atome durch Heteroatome 0, N oder S, ersetzt sind;
a 1, 2, 3, 4, 5, 6, 7, 8, 9 oder 10;
R(6) H, Aryl, Alkyl mit 1, 2, 3, 4, 5, 6, 7, 8, 9, 10, 11 oder 12 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch Heteroatome O, NH, S, ersetzt sind;
a 1, 2, 3, 4, 5, 6, 7, 8, 9 oder 10;
E Sauerstoff oder Schwefel;
X Sauerstoff oder Schwefel;
Y [CR(7)₂]₁₋₄, O, S oder NH;
R(7) Wasserstoff, Alkyl mit 1, 2, 3 oder 4 C-Atomen, F, Cl oder Br;
Aryl Phenyl, Thienyl, Furyl, Pyrrolyl, Thiazolyl, Naphthyl oder Pyridyl;
wobei der Cycloalkylrest zusätzlich einen Alkylsubstituenten tragen kann und der Arylrest jeweils durch ein bis drei Substituenten wie Alkyl mit 1 oder 2 C-Atomen, Alkoxy mit 1 oder 2 C-Atomen, Cl, Br oder F substituiert sein känn.
sowie ihre pharmazeutisch verträglichen Salze.

2. Verbindung der Formel I nach Anspruch 1, dadurch gekennzeichnet, daß darin bedeuten:
R(1) Wasserstoff, Alkyl mit 1, 2, 3, 4, 5, 6 oder 7 C-Atomen oder Cycloalkyl mit 3, 4, 5, 6 oder 7 C-Atomen,
R(2) Wasserstoff, F, Cl, Cycloalkyl mit 3, 4, 5 oder 6 C-Atomen oder eine C_{b}H_{2b+1}-Gruppe, in der 1, 2, 3 oder 4 CH₂-Gruppen durch O, NH oder S ersetzt sein können;
b 1, 2, 3, 4, 5, 6, 7 oder 8;
R(3) Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 CH₂-Gruppen durch O, NH oder S ersetzt sind;
a 1, 2, 3, 4, 5, 6, 7, 8, 9 oder 10;
oder
R(3) und R(4) gemeinsam eine C_{b}H_{2b}-Gruppe, in der eine der CH₂-Gruppen durch ein Heteroatom ersetzt sein kann;
b 1, 2, 3, 4, 5, 6, 7 oder 8;
R(4), R(5) und R(6) unabhängig voneinander Wasserstoff, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch Heteroatome O, NH, S, ersetzt sind;
a 1, 2, 3, 4, 5, 6, 7, 8, 9 oder 10;
E Sauerstoff oder Schwefel;
X Sauerstoff oder Schwefel;
Y [CR(7)₂]₁₋₃, O, S oder NH;
R(7) Wasserstoff, Alkyl mit 1, 2, 3 oder 4 C-Atomen, F, Cl oder Br;
Aryl Phenyl, Thienyl, Furyl, Pyrrolyl, Thiazolyl, Naphthyl oder Pyridyl;
wobei der Cycloalkylrest und der Arylrest wie in Anspruch 1 definiert substituiert sein können;
sowie ihre pharmazeutisch verträglichen Salze.

3. Verbindung der Formel I nach einem oder mehreren der Ansprüche 1 und 2, dadurch gekennzeichnet, daß darin bedeuten:
R(1) Wasserstoff, Alkyl mit 1, 2, 3 oder 4 C-Atomen oder Cycloalkyl mit 3, 4, 5, 6 oder 7 C-Atomen;
R(2) F, Cl oder eine C_{b}H_{2b+1}-Gruppe, in der 1, 2, 3 oder 4 CH₂-Gruppen durch O, NH oder S ersetzt sein können;
b 1, 2, 3, 4, 5, 6, 7 oder 8;
R(3) Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen, eine (CₐH₂ₐ₊₁)-Gruppe, in der 1, 2, 3 oder 4 CH₂-Gruppen durch O, NH oder S ersetzt sind;
a 1, 2, 3, 4, 5, 6, 7, 8, 9 oder 10;
oder
R(3) und R(4) gemeinsam eine C_{b}H_{2b}-Gruppe, in der eine der CH₂-Gruppen durch O, NH oder S ersetzt sein kann;
b 1, 2, 3, 4, 5, 6, 7 oder 8;
R(4), R(5) und R(6) Wasserstoff, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch O, NH oder S ersetzt sind;
a 1, 2, 3, 4, 5, 6, 7, 8, 9 oder 10;
E Sauerstoff oder Schwefel;
X Sauerstoff oder Schwefel;
Y [CR(7)₂]₁₋₃, O, S oder NH;
R(7) Wasserstoff, Alkyl mit 1, 2, 3 oder 4 C-Atomen, F, Cl oder Br;
Aryl Phenyl, Thienyl, Furyl, Pyrrolyl, Thiazolyl, Naphthyl oder Pyridyl;
wobei der Cycloalkylrest und der Arylrest wie in Anspruch 1 definiert substituiert sein können;
sowie ihre pharmazeutisch verträglichen Salze.

4. Verbindung der Formel I nach einem oder mehreren der Ansprüche 1 bis 3, dadurch gekennzeichnet, daß eine für R(2) stehende C_{b}H_{2b+1}-Gruppe, in der 1, 2, 3 oder 4 C-Atome durch Heteroatome O, N oder S ersetzt sein können, für Alkyl mit 1, 2, 3, 4, 5 oder 6 C-Atomen, Alkoxy mit 1, 2, 3, 4, 5 oder 6 C-Atomen oder Mercaptoalkyl mit 1, 2, 3, 4, 5 oder 6 C-Atomen steht; sowie ihre pharmazeutisch verträglichen Salze.

5. Verbindung der Formel I nach einem oder mehreren der Ansprüche 1 bis 4, dadurch gekennzeichnet, daß darin bedeuten:
R(1) Wasserstoff, Alkyl mit 1, 2, 3 oder 4 C-Atomen, Cycloalkyl mit 3 oder 4 C-Atomen;
R(2) Methoxy oder Ethoxy;
R(3) Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 CH₂-Gruppen durch O, NH, S ersetzt sind;
a 1, 2, 3, 4, 5, 6, 7, 8, 9 oder 10;
oder
R(3) und R(4) gemeinsam eine C_{b}H_{2b}-Gruppe, in der eine der CH₂-Gruppen durch O, NH oder S ersetzt sein kann;
b 1, 2, 3, 4, 5, 6, 7 oder 8;
R(4), R(5) und R(6) unabhängig voneinander Wasserstoff, Aryl, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch O, NH oder S ersetzt sind;
a 1, 2, 3, 4, 5, 6, 7, 8, 9 oder 10;
E Schwefel;
X Sauerstoff;
Y [CR(7)₂]₁₋₃, O, S oder NH;
R(7) Wasserstoff, Alkyl mit 1, 2, 3 oder 4 C-Atomen, F, Cl oder Br
Aryl Phenyl, Thienyl, Furyl, Pyrrolyl, Thiazolyl, Naphthyl oder Pyridyl;
wobei der Cycloalkylrest und der Arylrest wie in Anspruch 1 definiert substituiert sein können;
sowie ihre pharmazeutisch verträglichen Salze.

6. Verbindung der Formel I nach einem oder mehreren der Ansprüche 1 bis 5, dadurch gekennzeichnet, daß darin bedeuten:
R(1) Wasserstoff, Alkyl mit 1, 2, 3 oder 4 C-Atomen oder Cycloalkyl mit 3 oder 4 C-Atomen;
R(2) Methoxy oder Ethoxy;
R(3) Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 CH₂-Gruppen durch O, NH oder S ersetzt sind;
a 1, 2, 3, 4, 5, 6, 7, 8, 9 oder 10;
oder
R(3) und R(4) gemeinsam eine C_{b}H_{2b}-Gruppe, in der eine der CH₂-Gruppen durch O, S oder NH ersetzt sein kann;
b 1, 2, 3, 4, 5, 6, 7 oder 8;
R(4), R(5) und R(6) unabhängig voneinander Wasserstoff, Aryl, Alkyl mit 1, 2, 3, 4, 5, 6, 7, 8; 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch O, NH oder S ersetzt sind;
a 1, 2, 3, 4, 5, 6, 7, 8, 9 oder 10;
E Schwefel;
X Sauerstoff;
Y ICR(7)₂]₁₋₃;
R(7) Wasserstoff, Alkyl mit 1, 2, 3 oder 4 C-Atomen, F, Cl oder Br;
Aryl Phenyl, Thienyl, Furyl, Pyrrolyl, Thiazolyl, Naphthyl oder Pyridyl;
wobei der Cycloalkyrest und der Arylrest wie in Anspruch 1 definiert substituiert sein können;
sowie ihre pharmazeutisch verträglichen Salze.

7. Verbindung der Formel I nach einem oder mehreren der Ansprüche 1 bis 6, dadurch gekennzeichnet, daß darin bedeuten:
R(1) Wasserstoff, Alkyl mit 1, 2, 3 oder 4 C-Atomen oder Cycloalkyl mit 3 oder 4 C-Atomen;
R(2) Methoxy oder Ethoxy;
R(3) Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 CH₂-Gruppen durch O, S oder NH ersetzt sind;
a 1, 2, 3, 4, 5, 6, 7, 8, 9 oder 10;
oder
R(3) und R(4) gemeinsam einen C_{b}H_{2b}-Gruppe, in der eine der CH₂-Gruppen durch O, S oder NH ersetzt sein kann;
b 1, 2, 3, 4, 5, 6, 7 oder 8;
R(4), R(5) und R(6) unabhängig voneinander Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch O, NH oder S ersetzt sind;
a 1, 2, 3, 4, 5, 6, 7, 8, 9 oder 10;
E Schwefel;
X Sauerstoff;
Y CR(7)₂;
R(7) Wasserstoff, Alkyl mit 1, 2, 3 oder 4 C-Atomen, F oder Cl;
wobei der Cycloalkylrest wie in Anspruch 1 definiert substituiert sein kann ;
sowie ihre pharmazeutisch verträglichen Salze.

8. Verbindung der Formel I nach einem oder mehreren der Ansprüche 1 bis 7, dadurch gekennzeichnet, daß darin bedeuten:
R(1) Wasserstoff, Alkyl mit 1, 2, 3 oder 4 C-Atomen oder Cycloalkyl mit 3 oder 4 C-Atomen;
R(2) Methoxy oder Ethoxy;
R(3) Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 CH₂-Gruppen durch O, NH oder S ersetzt sind;
a 1, 2, 3, 4, 5, 6, 7, 8, 9 oder 10;
oder
R(3) und R(4) gemeinsam eine C_{b}H_{2b}-Gruppe, in der eine der CH₂-Gruppen durch O, NH oder S ersetzt sein kann;
b 1, 2, 3, 4, 5, 6, 7 oder 8;
R(4) und R(5) unabhängig voneinander Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (CₐH₂ₐ₊₁)-Gruppe, in der 1, 2, 3 oder 4 C-Atome durch O, NH oder S ersetzt sind;
a 1, 2, 3, 4, 5, 6, 7, 8, 9 oder 10;
R(6) Aryl;
E Schwefel;
X Sauerstoff;
Y CR(7)₂;
R(7) Wasserstoff, Alkyl mit 1 oder 2 C-Atomen, F oder Cl
Aryl Phenyl, Thienyl, Furyl, Pyrrolyl, Thiazolyl, Naphthyl oder Pyridyl;
wobei der Cycloalkylrest und der Arylrest wie in Anspruch 1 definiert substituiert sein können ;
sowie ihre pharmazeutisch verträglichen Salze.

9. Verfahren zum Herstellen einer Verbindung der Formel I nach einem oder mehreren der Ansprüche 1 bis 8, dadurch gekennzeichnet, daß man
(a) ein Sulfonamid der Formel II 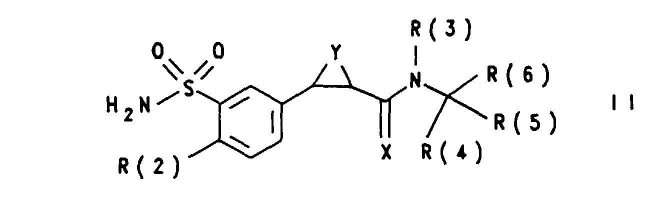 oder dessen Salz der Formel III 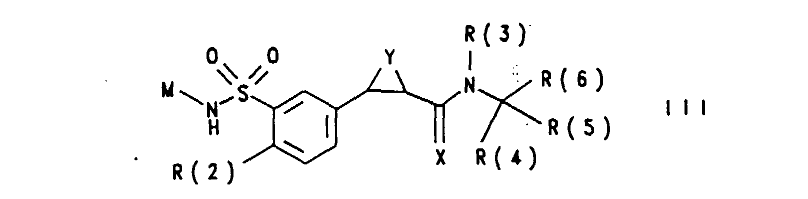 mit einem R(1)-substituierten Isocyanat der Formel IV
R(1) - N = C = O IV
zu einem substituierten Benzolsulfonylharnstoff I a umsetzt 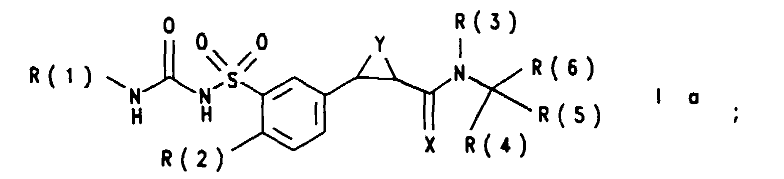 oder daß man
(b) einen Benzolsulfonylharnstoff I a aus einem aromatischen Benzolsulfonamid II oder dessen Salz III mit einem R(1)-substituierten Trichloracetamid der Formel V
Cl₃C-(CO)-NHR(1) V
darstellt; oder daß man
(c) einen Benzolsulfonylthioharnstoff I b 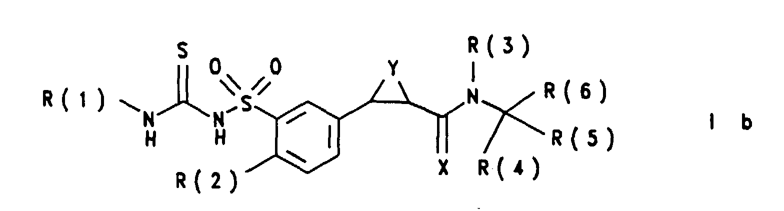 aus einem Benzolsulfonamid II oder dessen Salz III und einem R(1)-substituierten Thioisocyanat VI
R(1) - N = C = S VI
darstellt; oder daß man
(d) einen substituierten Benzolsulfonylharnstoff der Formel I a durch Umwandlungsreaktion von einem Benzolsulfonylthioharnstoff der Struktur I b darstellt; oder daß man
(e) einen Benzolsulfonylharnstoff I a durch Umsetzung eines Amins der Formel R(1)-NH₂ mit einem Benzolsulfonylisocyanat der Formel VII 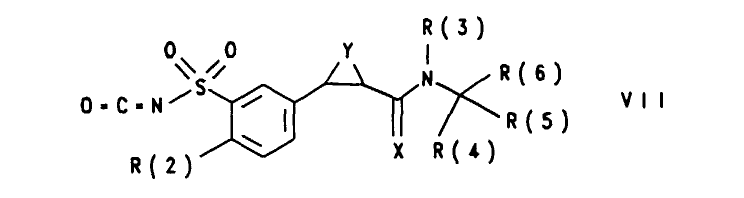 herstellt; oder daß man
(f) einen Benzolsulfonylthioharnstoff I b durch Umsetzung eines Amins der Formel R(1)-NH₂ mit einem Benzolsulfonylisothiocyanat der Formel VIII 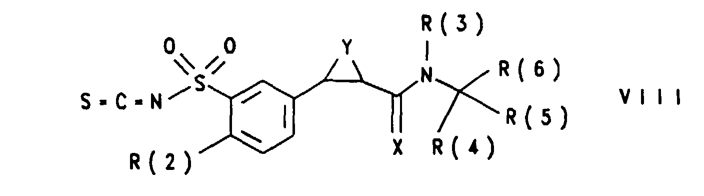 herstellt;
oder daß man
(g) einen Benzolsulfonylharnstoff Ia aus einem Benzolsulfonylharnstoff der Formel IX a 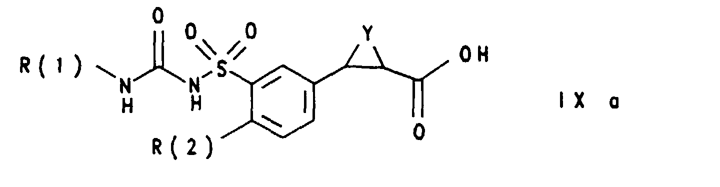 und R(3)R(4)NH mittels wasserentziehender Mittel oder Aktivierung mittels Carbonsäurehalogenide bzw. Bildung gemischter Anhydride darstellt; oder daß man
(h) einen Benzolsulfonylthioharnstoff I b aus einem Benzolsulfonylthioharnstoff der Formel IX b 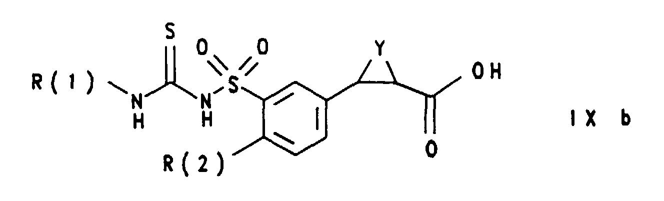 und R(3)R(4)NH mittels wasserentziehender Mittel oder Aktivierung mittels Carbonsäurehalogenide bzw. Bildung gemischter Anhydride darstellt.

10. Verwendung einer Verbindung der Formel I nach einem oder mehreren der Ansprüche 1 bis 8 oder eines pharmazeutisch verträglichen Salzes davon zur Herstellung eines Medikaments zur Behandlung von Herzrhythmusstörungen.

11. Verwendung einer Verbindung der Formel I nach einem oder mehreren der Ansprüche 1 bis 8 oder eines pharmazeutisch verträglichen Salzes davon zur Herstellung eines Medikaments zur Verhinderung des plötzlichen Herztods.

12. Verwendung einer Verbindung der Formel I nach einem oder mehreren der Ansprüche 1 bis 8 oder eines pharmazeutisch verträglichen Salzes davon zur Herstellung eines Medikaments zur Behandlung von ischämischen Zuständen des Herzens.

13. Verwendung einer Verbindung der Formel I nach einem oder mehreren der Ansprüche 1 bis 8 oder eines pharmazeutisch verträglichen Salzes davon zur Herstellung eines Medikaments zur Behandlung der geschwächten Herzkraft.

14. Verwendung einer Verbindung der Formel I nach einem oder mehreren der Ansprüche 1 bis 8 oder eines pharmazeutisch verträglichen Salzes davon zur Herstellung eines Medikaments zur Verbesserung der Herzfunktion nach Herztransplantation.

15. Heilmittel, gekennzeichnet durch eine wirksame Menge an einer Verbindung der Formel I nach einem oder mehreren der Ansprüche 1 bis 8 oder eines pharmazeutisch verträglichen Salzes davon.

## Claims

1. A substituted benzenesulfonylurea or -thiourea of the formula I 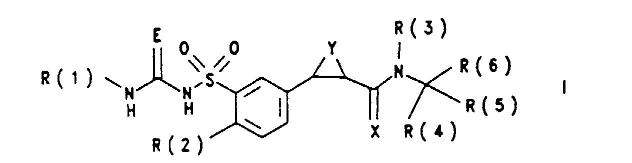 in which:
R(1) is hydrogen, alkyl having 1, 2, 3, 4, 5, 6 or 7 carbon atoms or cycloalkyl having 3, 4, 5, 6 or 7 carbon atoms;
R(2) is hydrogen, F, Cl, cycloalkyl having 3, 4, 5 or 6 carbon atoms or a C_{b}H_{2b+1} group in which 1, 2, 3 or 4 carbon atoms can be replaced by heteroatoms O, N or S;
b is 1, 2, 3, 4, 5, 6, 7 or 8;
R(3) is H, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 carbon atoms are replaced by heteroatoms, e.g. O, NH or S;
a is 1, 2, 3, 4, 5, 6, 7, 8, 9 or 10;
R(4) is H, aryl, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 carbon atoms are replaced by heteroatoms, e.g. O, NH or S;
a is 1, 2, 3, 4, 5, 6, 7, 8, 9 or 10;
or
R(3) and R(4) together are a C_{b}H_{2b} group in which one or more of the CH₂ groups can be replaced by heteroatoms, e.g. by O, S or NH;
b is 1, 2, 3, 4, 5, 6, 7 or 8;
R(5) is H, aryl, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 carbon atoms are replaced by heteroatoms O, N or S;
a is 1, 2, 3, 4, 5, 6, 7, 8, 9 or 10;
R(6) is H, aryl, alkyl having 1, 2, 3, 4, 5, 6, 7, 8, 9, 10, 11 or 12 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 carbon atoms are replaced by heteroatoms O, NH or S;
a is 1, 2, 3, 4, 5, 6, 7, 8, 9 or 10;
E is oxygen or sulfur;
X is oxygen or sulfur;
Y is [CR(7)₂]₁₋₄, O, S or NH;
R(7) is hydrogen, alkyl having 1, 2, 3 or 4 carbon atoms, F, Cl or Br;
aryl is phenyl, thienyl, furyl, pyrrolyl, thiazolyl, naphthyl or pyridyl;
it being possible for the cycloalkyl radical to additionally carry an alkyl substituent and for the aryl radical to be substituted in each case by one to three substituents such as alkyl having 1 or 2 carbon atoms, alkoxy having 1 or 2 carbon atoms, Cl, Br or F;
or its pharmaceutically tolerable salts.

2. A compound of the formula I as claimed in claim 1, wherein:
R(1) is hydrogen, alkyl having 1, 2, 3, 4, 5, 6 or 7 carbon atoms or cycloalkyl having 3, 4, 5, 6 or 7 carbon atoms,
R(2) is hydrogen, F, Cl, cycloalkyl having 3, 4, 5 or 6 carbon atoms or a C_{b}H_{2b+1} group in which 1, 2, 3 or 4 CH₂ groups can be replaced by O, NH or S;
b is 1, 2, 3, 4, 5, 6, 7 or 8;
R(3) is hydrogen, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which I, 2, 3 or 4 CH₂ groups are replaced by O, NH or S;
a is 1, 2, 3, 4, 5, 6, 7, 8, 9 or 10;
or
R(3) and R(4) together are a C_{b}H_{2b} group in which one of the CH₂ groups can be replaced by a heteroatom;
b is 1, 2, 3, 4, 5, 6, 7 or 8;
R(4), R(5) and R(6) independently of one another are hydrogen, aryl, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 carbon atoms are replaced by heteroatoms O, NH or S;
a is 1, 2, 3, 4, 5, 6, 7, 8, 9 or 10;
E is oxygen or sulfur;
X is oxygen or sulfur;
Y is [OR(7)₂]₁₋₃, O, S or NH;
R(7) is hydrogen, alkyl having 1, 2, 3 or 4 carbon atoms, F, Cl or Br;
aryl is phenyl, thienyl, furyl, pyrrolyl, thiazolyl, naphthyl or pyridyl;
it being possible for the cycloalkyl radical and the aryl radical to be substituted as defined in claim 1;
or its pharmaceutically tolerable salts.

3. A compound of the formula I as claimed in one or more of claims 1 and 2, wherein:
R(1) is hydrogen, alkyl having 1, 2, 3 or 4 carbon atoms or cycloalkyl having 3, 4, 5, 6 or 7 carbon atoms;
R(2) is F, Cl or a C_{b}H_{2b+1} group in which 1, 2, 3 or 4 CH₂ groups can be replaced by O, NH or S;
b is 1, 2, 3, 4, 5, 6, 7 or 8;
R(3) is hydrogen, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 CH₂ groups are replaced by O, NH or S;
a is 1, 2, 3, 4, 5, 6, 7, 8, 9 or 10;
or
R(3) and R(4) together are a C_{b}H_{2b} group in which one of the CH₂ groups can be replaced by O, NH or S;
b is 1, 2, 3, 4, 5, 6, 7 or 8;
R(4), R(5) and R(6) are hydrogen, aryl, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 carbon atoms are replaced by O, NH or S;
a is 1, 2, 3, 4, 5, 6, 7, 8, 9 or 10;
E is oxygen or sulfur;
X is oxygen or sulfur;
Y is [CR(7)₂]₁₋₃, O, S or NH;
R(7) is hydrogen, alkyl having 1, 2, 3 or 4 carbon atoms, F, Cl or Br;
aryl is phenyl, thienyl, furyl, pyrrolyl, thiazolyl, naphthyl or pyridyl;
it being possible for the cycloalkyl radical and the aryl radical to be substituted as defined in claim 1;
or its pharmaceutically tolerable salts.

4. A compound of the formula I as claimed in one or more of claims 1 to 3, wherein a C_{b}H_{2b+1} group representing R(2), in which 1, 2, 3 or 4 carbon atoms can be replaced by heteroatoms O, N or S, is alkyl having 1, 2, 3, 4, 5 or 6 carbon atoms, alkoxy having 1, 2, 3, 4, 5 or 6 carbon atoms or mercaptoalkyl having 1, 2, 3, 4, 5 or 6 carbon atoms;
or its pharmaceutically tolerable salts.

5. A compound of the formula I as claimed in one or more of claims 1 to 4, wherein:
R(1) is hydrogen, alkyl having 1, 2, 3 or 4 carbon atoms or cycloalkyl having 3 or 4 carbon atoms;
R(2) is methoxy or ethoxy;
R(3) is hydrogen, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 OR 4 CH₂ groups are replaced by O, NH or S; a is 1, 2, 3, 4, 5, 6, 7, 8, 9 or 10;
or
R(3) and R(4) together are a C_{b}H_{2b} group in which one of the CH₂ groups can be replaced by O, NH or S;
b is 1, 2, 3, 4, 5, 6, 7 or 8;
R(4), R(5) and R(6) independently of one another are hydrogen, aryl, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 carbon atoms are replaced by O, NH or S;
a is 1, 2, 3, 4, 5, 6, 7, 8, 9 or 10;
E is sulfur;
X is oxygen;
Y is [CR(7)₂]₁₋₃, O, S or NH;
R(7) is hydrogen, alkyl having 1, 2, 3 or 4 carbon atoms, F, Cl or Br;
aryl is phenyl, thienyl, furyl, pyrrolyl, thiazolyl, naphthyl or pyridyl;
it being possible for the cycloalkyl radical and the aryl radical to be substituted as defined in claim 1;
or its pharmaceutically tolerable salts.

6. A compound of the formula I as claimed in one or more of claims 1 to 5, wherein:
R(1) is hydrogen, alkyl having 1, 2, 3 or 4 carbon atoms or cycloalkyl having 3 or 4 carbon atoms;
R(2) is methoxy or ethoxy;
R(3) is hydrogen, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 CH₂ groups are replaced by O, NH or S; a is 1, 2, 3, 4, 5, 6, 7, 8, 9 or 10;
or
R(3) and R(4) together are a C_{b}H_{2b} group in which one of the CH₂ groups can be replaced by O, S or NH;
b is 1, 2, 3, 4, 5, 6, 7 or 8;
R(4) , R(5) and R(6) independently of one another are hydrogen, aryl, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 carbon atoms are replaced by O, NH or S;
a is 1, 2, 3, 4, 5, 6, 7, 8, 9 or 10;
E is sulfur;
X is oxygen;
Y is [CR(7)₂]₁₋₃;
R(7) is hydrogen, alkyl having 1, 2, 3 or 4 carbon atoms, F, Cl or Br;
aryl is phenyl, thienyl, furyl, pyrrolyl, thiazolyl, naphthyl or pyridyl;
it being possible for the cycloalkyl radical and the aryl radical to be substituted as defined in claim 1;
or its pharmaceutically tolerable salts.

7. A compound of the formula I as claimed in one or more of claims 1 to 6, wherein:
R(1) is hydrogen, alkyl having 1, 2, 3 or 4 carbon atoms or cycloalkyl having 3 or 4 carbon atoms;
R(2) is methoxy or ethoxy;
R(3) is hydrogen, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 CH₂ groups are replaced by O, S or NH;
a is 1, 2, 3, 4, 5, 6, 7, 8, 9 or 10;
or
R(3) and R(4) together are a C_{b}H_{2b} group in which one of the CH₂ groups can be replaced by O, S or NH;
b is 1, 2, 3, 4, 5, 6, 7 or 8;
R(4) , R(5) and R(6) independently of one another are hydrogen, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 carbon atoms are replaced by O, NH or S;
a is 1, 2, 3, 4, 5, 6, 7, 8, 9 or 10;
E is sulfur;
X is oxygen;
Y is CR(7)₂;
R(7) is hydrogen or alkyl having 1, 2, 3 or 4 carbon atoms, F or Cl;
it being possible for the cycloalkyl radical to be substituted as defined in claim 1;
or its pharmaceutically tolerable salts.

8. A compound of the formula I as claimed in one or more of claims 1 to 7, wherein:
R(1) is hydrogen, alkyl having 1, 2, 3 or 4 carbon atoms or cycloalkyl having 3 or 4 carbon atoms;
R(2) is methoxy or ethoxy;
R(3) is hydrogen, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 CH₂ groups are replaced by O, NH or S;
a is 1, 2, 3, 4, 5, 6, 7, 8, 9 or 10;
or
R(3) and R(4) together are a C_{b}H_{2b} group in which one of the CH₂ groups can be replaced by O, NH or S;
b is 1, 2, 3, 4, 5, 6, 7 or 8;
R(4) and R(5) independently of one another are hydrogen, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (CₐH₂ₐ₊₁) group in which 1, 2, 3 or 4 carbon atoms are replaced by O, NH or S;
a is 1, 2, 3, 4, 5, 6, 7, 8, 9 or 10;
R(6) is aryl;
E is sulfur;
X is oxygen;
Y is CR(7)₂;
R(7) is hydrogen, alkyl having 1 or 2 carbon atoms, F or Cl;
aryl is phenyl, thienyl, furyl, pyrrolyl, thiazolyl, naphthyl or pyridyl;
it being possible for the cycloalkyl radical and the aryl radical to be substituted as defined in claim 1;
or its pharmaceutically tolerable salts.

9. A process for preparing a compound of the formula I as claimed in one or more of claims 1 to 8, which comprises
(a) reacting a sulfonamide of the formula II 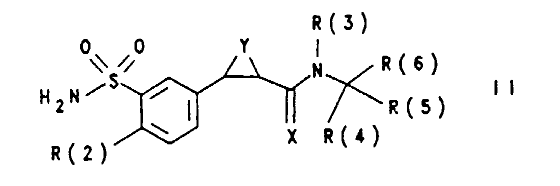 or its salt of the formula III 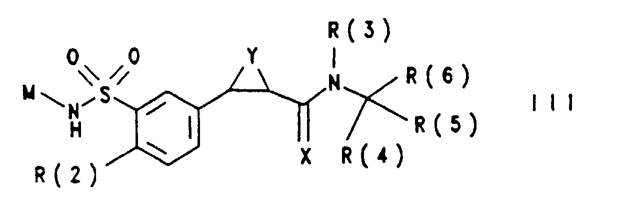 with an R(1)-substituted isocyanate of the formula IV
R(1)-N=C=O IV
to give a substituted benzenesulfonylurea I a 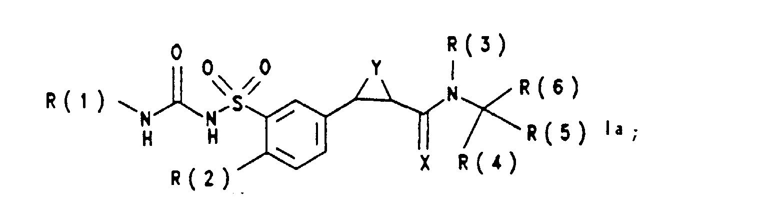 or
(b) preparing a benzenesulfonylurea I a from an aromatic benzenesulfonamide II or its salt III using an R(1)-substituted trichloroacetamide of the formula V
Cl₃C-(CO)-NHR(1) V;
or
(c) preparing a benzenesulfonylthiourea I b 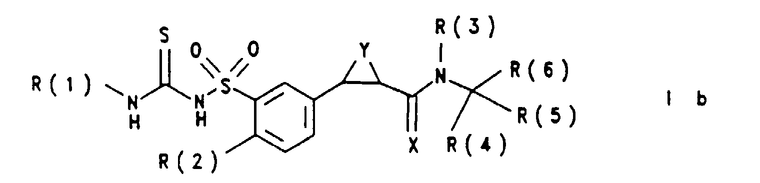 from a benzenesulfonamide II or its salt III and an R(1)-substituted isothiocyanate VI
R(1)-N=C=S VI;
or
(d) preparing a substituted benzenesulfonylurea of the formula I a by a conversion reaction of a benzenesulfonylthiourea of the structure Ib; or
(e) preparing a benzenesulfonylurea I a by reaction of an amine of the formula R(1)-NH₂ with a benzenesulfonyl isocyanate of the formula VII 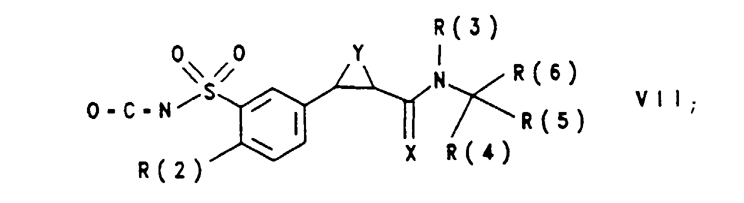 or
(f) preparing a benzenesulfonylthiourea I b by reaction of an amine of the formula R(1)-NH₂ with a benzenesulfonyl isothiocyanate of the formula VIII 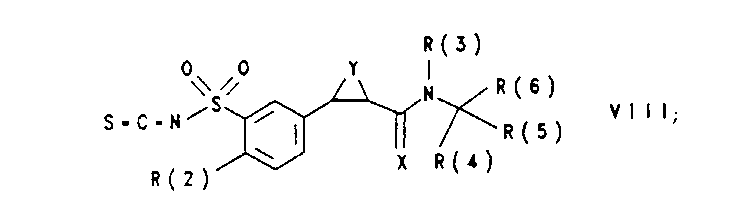 or
(g) preparing a benzenesulfonylurea I a from a benzenesulfonylurea of the formula IX a 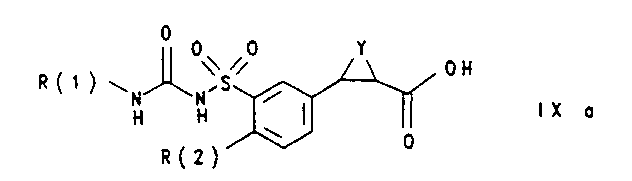 and R(3)R(4)NH by means of dehydrating agents or activation by means of carbonyl halides or formation of mixed anhydrides; or
(h) preparing a benzenesulfonylthiourea I b from a benzenesulfonylthiourea of the formula IX b 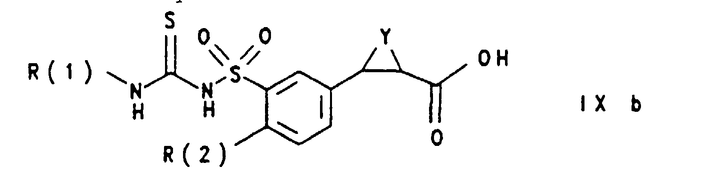 and R(3)R(4)NH by means of dehydrating agents or activation by means of carbonyl halides or formation of mixed anhydrides.

10. The use of a compound of the formula I as claimed in one or more of claims I to 8 or of a pharmaceutically tolerable salt thereof for the production of a medicament for the treatment of cardiac arrhythmias.

11. The use of a compound of the formula I as claimed in one or more of claims 1 to 8 or of a pharmaceutically tolerable salt thereof for the production of a medicament for the prevention of sudden heart death.

12. The use of a compound of the formula I as claimed in one or more of claims I to 8 or of a pharmaceutically tolerable salt thereof for the production of a medicament for the treatment of ischemic conditions of the heart.

13. The use of a compound of the formula I as claimed in one or more of claims 1 to 8 or of a pharmaceutically tolerable salt thereof for the production of a medicament for the treatment of weakened cardiac power.

14. The use of a compound of the formula I as claimed in one or more of claims 1 to 8 or of a pharmaceutically tolerable salt thereof for the production of a medicament for the improvement of heart function after heart transplantation.

15. A medicament comprising an effective amount of a compound of the formula I as claimed in one or more of claims 1 to 8 or of a pharmaceutically tolerable salt thereof.

## Revendications

1. Benzènesulfonylurées et -thio-urées substituées de formule I 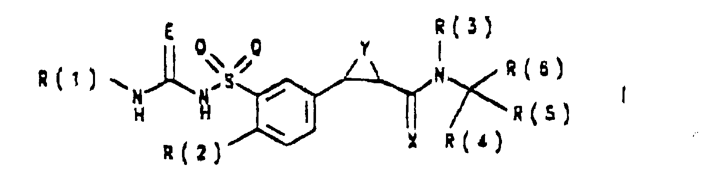 dans laquelle
R(1) représente un atome d'hydrogène ou un groupe alkyle ayant 1, 2, 3, 4, 5, 6 ou 7 atomes de carbone, ou cycloalkyle ayant 3, 4, 5, 6 ou 7 atomes de carbone;
R(2) représente un atome d'hydrogène, F, Cl, un groupe cycloalkyle ayant 3, 4, 5 ou 6 atomes de carbone ou un groupe C_{b}H_{2b+1} dans lequel 1, 2, 3 ou 4 atomes de carbone peuvent être remplacés par des hétéroatomes O, N ou S;
b étant 1, 2, 3, 4, 5, 6, 7 ou 8;
R(3) représente H ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 atomes de carbone sont remplacés par des hétéroatomes, par exemple O, NH, S;
a étant 1, 2, 3, 4, 5, 6, 7, 8, 9 ou 10;
R(4) représente H ou un groupe aryle, alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 atomes de carbone sont remplacés par des hétéroatomes, par exemple O, NH, S;
a étant 1, 2, 3, 4, 5, 6, 7, 8, 9 ou 10;
ou
R(3) et R(4) forment ensemble un groupe C_{b}H_{2b}, dans lequel un ou plusieurs des groupes CH₂ peut(peuvent) être remplacé(s) par des hétéroatomes, par exemple O, S ou NH;
b étant 1, 2, 3, 4, 5, 6, 7 ou 8;
R(5) représente H ou un groupe aryle, alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 atomes de carbone sont remplacés par des hétéroatomes O, N ou S;
a étant 1, 2, 3, 4, 5, 6, 7, 8, 9 ou 10;
R(6) représente H ou un groupe aryle, alkyle ayant 1, 2, 3, 4, 5, 6, 7, 8, 9, 10, 11 ou 12 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 atomes de carbone sont remplacés par des hétéroatomes O, NH, S;
a étant 1, 2, 3, 4, 5, 6, 7, 8, 9 ou 10;
E représente un atome d'oxygène ou de soufre;
X représente un atome d'oxygène ou de soufre;
Y représente un groupe [CR(7)₂]₁₋₄, O, S ou NH;
R(7) étant un atome d'hydrogène ou un groupe alkyle ayant 1, 2, 3 ou 4 atomes de carbone, F, Cl ou Br;
aryle étant le groupe phényle, thiényle, furyle, pyrrolyle, thiazolyle, naphtyle ou pyridyle; le radical cycloalkyle pouvant porter en outre un substituant alkyle, et le groupe aryle pouvant, dans chaque, cas être substitué par un à trois substituants, tels que des groupes alkyle ayant 1 ou 2 atomes de carbone, des groupes alcoxy ayant 1 ou 2 atomes de carbone, Cl, Br ou F.
ainsi que leurs sels pharmaceutiquement acceptables.

2. Composé de formule I selon la revendication 1, caractérisé en ce que dans cette formule:
R(1) représente un atome d'hydrogène ou un groupe alkyle ayant 1, 2, 3, 4, 5, 6 ou 7 atomes de carbone, ou cycloalkyle ayant 3, 4, 5, 6 ou 7 atomes de carbone;
R(2) représente un atome d'hydrogène, F, Cl, un groupe cycloalkyle ayant 3, 4, 5 ou 6 atomes de carbone ou un groupe C_{b}H_{2b+1} dans lequel 1, 2, 3 ou 4 groupes CH₂ peuvent être remplacés par O, NH ou S;
b étant1, 2, 3, 4, 5, 6, 7 ou 8;
R(3) représente un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 groupes CH₂ sont remplacés par O, NH, S;
a étant 1, 2, 3, 4, 5, 6, 7, 8, 9 ou 10;
ou
R(3) et R(4) forment ensemble un groupe C_{b}H_{2b}, dans lequel l'un des groupes CH₂ peut être remplacé par un hétéroatome;
b étant 1, 2, 3, 4, 5, 6, 7 ou 8;
R(4), R(5) et R(6) représentent, indépendamment les uns des autres, un atome d'hydrogène ou un groupe aryle, alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 atomes de carbone sont remplacés par des hétéroatomes O, NH, S;
a étant 1, 2, 3, 4, 5, 6, 7, 8, 9 ou 10;
E représente un atome d'oxygène ou de soufre;
X représente un atome d'oxygène ou de soufre;
Y représente un groupe [CR(7)₂]₁₋₃, O, S ou NH; R(7) étant un atome d'hydrogène ou un groupe alkyle ayant 1, 2, 3 ou 4 atomes de carbone, F, Cl ou Br;
aryle étant le groupe phényle, thiényle, furyle, pyrrolyle, thiazolyle, naphtyle ou pyridyle; le radical cycloalkyle et le radical aryle pouvant être substitués comme défini dans la revendication 1;
ainsi que ses sels pharmaceutiquement acceptables.

3. Composé de formule I selon une ou plusieurs des revendications 1 et 2, caractérisé en ce que dans cette formule:
R(1) représente un atome d'hydrogène ou un groupe alkyle ayant 1, 2, 3 ou 4 atomes de carbone, ou cycloalkyle ayant 3, 4, 5, 6 ou 7 atomes de carbone;
R(2) représente F, Cl ou un groupe C_{b}H_{2b+1} dans lequel 1, 2, 3 ou 4 groupes CH₂ peuvent être remplacés par O, NH ou S;
b étant 1, 2, 3, 4, 5, 6, 7 ou 8;
R(3) représente un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 groupes CH₂ sont remplacés par O, NH ou S;
a étant 1, 2, 3, 4, 5, 6, 7, 8, 9 ou 10;
ou
R(3) et R(4) forment ensemble un groupe C_{b}H_{2b}, dans lequel l'un des groupes CH₂ peut être remplacé par O, NH ou S ;
b étant 1, 2, 3, 4, 5, 6, 7 ou 8;
R(4), R(5) et R(6) représentent, indépendamment les uns des autres, un atome d'hydrogène ou un groupe aryle, alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 atomes de carbone sont remplacés par O, NH ou S;
a étant 1, 2, 3, 4, 5, 6, 7, 8, 9 ou 10;
E représente un atome d'oxygène ou de soufre;
X représente un atome d'oxygène ou de soufre;
Y représente un groupe [CR(7)₂]₁₋₃, O, S ou NH;
R(7) étant un atome d'hydrogène ou un groupe alkyle ayant 1, 2, 3 ou 4 atomes de carbone, F, Cl ou Br;
aryle étant le groupe phényle, thiényle, furyle, pyrrolyle, thiazolyle, naphtyle ou pyridyle; le radical cycloalkyle et le radical aryle pouvant être substitués comme défini dans la revendication 1;
ainsi que ses sels pharmaceutiquement acceptables.

4. Composé de formule I selon une ou plusieurs des revendications 1 à 3, caractérisé en ce qu'un groupe C_{b}H_{2b+1} représenté par R(2), dans lequel 1, 2, 3 ou 4 atomes de carbone peuvent être remplacés par des hétéroatomes O, N ou S, représente un groupe alkyle ayant 1, 2, 3, 4, 5 ou 6 atomes de carbone, alcoxy ayant 1, 2, 3, 4, 5 ou 6 atomes de carbone ou mercaptoalkyle ayant 1, 2, 3, 4, 5 ou 6 atomes de carbone;
ainsi que ses sels pharmaceutiquement acceptables.

5. Composé de formule I selon une ou plusieurs des revendications 1 à 4, caractérisé en ce que dans cette formule:
R(1) représente un atome d'hydrogène ou un groupe alkyle ayant 1, 2, 3 ou 4 atomes de carbone, ou cycloalkyle ayant 3 ou 4 atomes de carbone;
R(2) représente le groupe méthoxy ou éthoxy;
R(3) représente un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 groupes CH₂ sont remplacés par O, NH, S;
a étant 1, 2, 3, 4, 5, 6, 7, 8, 9 ou 10;
ou
R(3) et R(4) forment ensemble un groupe C_{b}H_{2b}, dans lequel l'un des groupes CH₂ peut être remplacé par O, NH ou S;
b étant 1, 2, 3, 4, 5, 6, 7 ou 8;
R(4), R(5) et R(6) représentent, indépendamment les uns des autres, un atome d'hydrogène ou un groupe aryle, alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 atomes de carbone sont remplacés par O, NH ou S;
a étant 1, 2, 3, 4, 5, 6, 7, 8, 9 ou 10;
E représente un atome de soufre;
X représente un atome d'oxygène;
Y représente un groupe [CR(7)₂]₁₋₃, O, S ou NH;
R(7) étant un atome d'hydrogène ou un groupe alkyle ayant 1, 2, 3 ou 4 atomes de carbone, F, Cl ou Br;
aryle étant le groupe phényle, thiényle, furyle, pyrrolyle, thiazolyle, naphtyle ou pyridyle; le radical cycloalkyle et le radical aryle pouvant être substitués comme défini dans la revendication 1;
ainsi que ses sels pharmaceutiquement acceptables.

6. Composé de formule I selon une ou plusieurs des revendications 1 à 5, caractérisé en ce que dans cette formule:
R(1) représente un atome d'hydrogène ou un groupe alkyle ayant 1, 2, 3 ou 4 atomes de carbone, ou cycloalkyle ayant 3 ou 4 atomes de carbone;
R(2) représente le groupe méthoxy ou éthoxy;
R(3) représente un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 groupes CH₂ sont remplacés par O, NH ou S;
a étant 1, 2, 3, 4, 5, 6, 7, 8, 9 ou 10;
ou
R(3) et R(4) forment ensemble un groupe C_{b}H_{2b}, dans lequel l'un des groupes CH₂ peut être remplacé par O, S ou NH;
b étant 1, 2, 3, 4, 5, 6, 7 ou 8;
R(4), R(5) et R(6) représentent, indépendamment les uns des autres, un atome d'hydrogène ou un groupe aryle, alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 atomes de carbone sont remplacés par O, NH ou S;
a étant 1, 2, 3, 4, 5, 6, 7, 8, 9 ou 10;
E représente un atome de soufre;
X représente un atome d'oxygène;
Y représente un groupe [CR(7)₂]₁₋₃;
R(7) étant un atome d'hydrogène ou un groupe alkyle ayant 1, 2, 3 ou 4 atomes de carbone, F, Cl ou Br;
aryle étant le groupe phényle, thiényle, furyle, pyrrolyle, thiazolyle, naphtyle ou pyridyle; le radical cycloalkyle et le radical aryle pouvant être substitués comme défini dans la revendication 1;
ainsi que ses sels pharmaceutiquement acceptables.

7. Composé de formule I selon une ou plusieurs des revendications 1 à 6, caractérisé en ce que dans cette formule:
R(1) représente un atome d'hydrogène ou un groupe alkyle ayant 1, 2, 3 ou 4 atomes de carbone, ou cycloalkyle ayant 3 ou 4 atomes de carbone;
R(2) représente le groupe méthoxy ou éthoxy;
R(3) représente un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 groupes CH₂ sont remplacés par O, S ou NH;
a étant 1, 2, 3, 4, 5, 6, 7, 8, 9 ou 10;
ou
R(3) et R(4) forment ensemble un groupe C_{b}H_{2b}, dans lequel l'un des groupes CH₂ peut être remplacé par O, S ou NH;
b étant 1, 2, 3, 4, 5, 6, 7 ou 8;
R(4), R(5) et R(6) représentent, indépendamment les uns des autres, un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 atomes de carbone sont remplacés par O, NH ou S;
a étant 1, 2, 3, 4, 5, 6, 7, 8, 9 ou 10;
E représente un atome de soufre;
X représente un atome d'oxygène;
Y représente un groupe CR(7)₂;
R(7) étant un atome d'hydrogène ou un groupe alkyle ayant 1, 2, 3 ou 4 atomes de carbone, F ou Cl;
le radical cycloalkyle pouvant être substitué comme défini dans la revendication 1;
ainsi que ses sels pharmaceutiquement acceptables.

8. Composé de formule I selon une ou plusieurs des revendications 1 à 7, caractérisé en ce que dans cette formule:
R(1) représente un atome d'hydrogène ou un groupe alkyle ayant 1, 2, 3 ou 4 atomes de carbone, ou cycloalkyle ayant 3 ou 4 atomes de carbone;
R(2) représente le groupe méthoxy ou éthoxy;
R(3) représente un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 groupes CH₂ sont remplacés par O NH ou S;
a étant 1, 2, 3, 4, 5, 6, 7, 8, 9 ou 10;
ou
R(3) et R(4) forment ensemble un groupe C_{b}H_{2b}, dans lequel l'un des groupes CH₂ peut être remplacé par O, NH ou S;
b étant 1, 2, 3, 4, 5, 6, 7 ou 8;
R(4) et R(5) représentent, indépendamment l'un de l'autre, un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou un groupe (CₐH₂ₐ₊₁) dans lequel 1, 2, 3 ou 4 atomes de carbone sont remplacés par O, NH ou S;
a étant 1, 2, 3, 4, 5, 6, 7, 8, 9 ou 10;
R(6) représente un groupe aryle;
E représente un atome de soufre;
X représente un atome d'oxygène;
Y représente un groupe CR(7)₂;
R(7) étant un atome d'hydrogène ou un groupe alkyle ayant 1 ou 2 atomes de carbone, F ou Cl;
aryle étant le groupe phényle, thiényle, furyle, pyrrolyle, thiazolyle, naphtyle ou pyridyle; le radical cycloalkyle et le radical aryle pouvant être substitués comme défini dans la revendication 1;
ainsi que ses sels pharmaceutiquement acceptables.

9. Procédé pour la préparation d'un composé I selon une ou plusieurs des revendications 1 à 8, caractérisé en ce que
(a) on fait réagir un sulfonamide de formule II 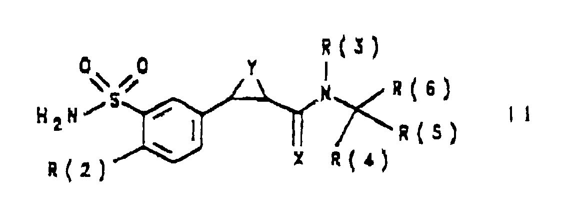 ou un sel de celui-ci, de formule III 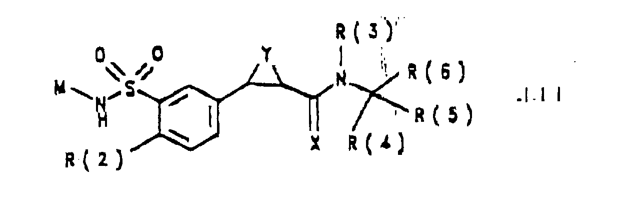 avec un isocyanate substitué par R(1), de formule IV
R(1)-N=C=O IV
pour aboutir à une benzènesulfonylurée substituée Ia, 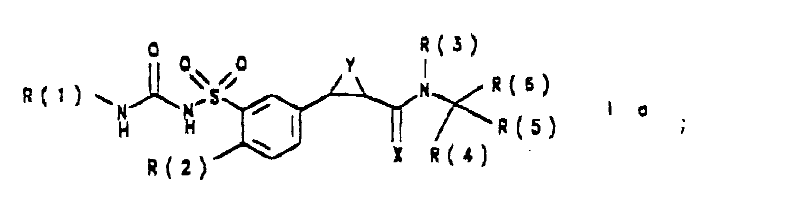 ou en ce que
(b) on prépare une benzènesulfonylurée Ia à partir d'un benzènesulfonamide aromatique II ou d'un sel III de celui-ci, avec un trichloracétamide substitué par R(1), de formule V
Cl₃C-(CO)-NHR(1) V;
ou en ce que
(c) on prépare une benzènesulfonylthio-urée Ib 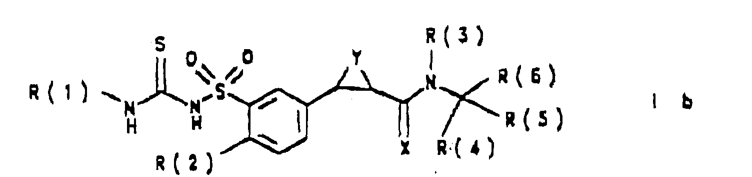 à partir d'un benzènesulfonamide II ou d'un de ses sels III et d'un thio-isocyanate VI substitué par R(1)
R(1)-N=C=S VI
ou en ce que
(d) on prépare une benzènesulfonylurée substituée de formule Ia par une réaction de conversion d'une benzènesulfonylthio-urée de structure Ib; ou en ce que
(e) on prépare une benzènesulfonylurée Ia par la réaction d'une amine de formule R(1)-NH₂ avec un benzènesulfonylisocyanate de formule VII 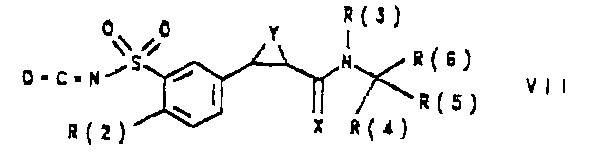 ou en ce que
(f) on prépare une benzènesulfonylthio-urée Ib par la réaction d'une amine de formule R(1)-NH₂ avec un benzène-sulfonylisothiocyanate de formule VIII 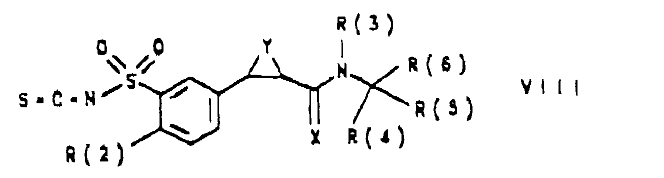 ou en ce que
(g) on prépare une benzènesulfonylurée Ia à partir d'une benzènesulfonylurée de formule IXa 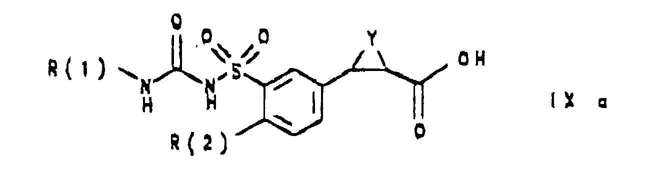 et R(3)R(4)NH, à l'aide d'agents de déshydratation ou par activation à l'aide d'halogénures de carbonyle ou formation d'anhydrides mixtes;
ou en ce que
(h) on prépare une benzènesulfonylthio-urée Ib à partir d'une benzènesulfonylthio-urée de formule IXb 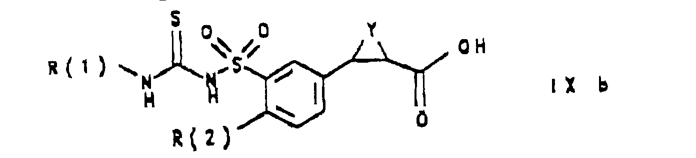 et R(3)R(4)NH, à l'aide d'agents de déshydratation ou par activation à l'aide d'halogénures de carbonyle ou formation d'anhydrides mixtes.

10. Utilisation d'un composé de formule I selon une ou plusieurs des revendications 1 à 8, ou d'un sel pharmaceutiquement acceptable de celui-ci, pour la fabrication d'un médicament destiné au traitement de troubles du rythme cardiaque.

11. Utilisation d'un composé de formule I selon une ou plusieurs des revendications 1 à 8, ou d'un sel pharmaceutiquement acceptable de celui-ci, pour la fabrication d'un médicament destiné à empêcher la mort subite par arrêt cardiaque.

12. Utilisation d'un composé de formule I selon une ou plusieurs des revendications 1 à 8, ou d'un sel pharmaceutiquement acceptable de celui-ci, pour la fabrication d'un médicament destiné au traitement d'états ischémiques du coeur.

13. Utilisation d'un composé de formule I selon une ou plusieurs des revendications 1 à 8, ou d'un sel pharmaceutiquement acceptable de celui-ci, pour la fabrication d'un médicament destiné au traitement de la force cardiaque affaiblie.

14. Utilisation d'un composé de formule I selon une ou plusieurs des revendications 1 à 8, ou d'un sel pharmaceutiquement acceptable de celui-ci, pour la fabrication d'un médicament destiné à l'amélioration de la fonction cardiaque après une greffe du coeur.

15. Médicament, caractérisé par une quantité efficace d'un composé de formule I selon une ou plusieurs des revendications 1 à 8, ou d'un sel pharmaceutiquement acceptable d'un tel composé.
